# EUROPÄISCHE PATENTSCHRIFT

(11) **EP 0 648 778 B1**
(45) Veröffentlichungstag und Bekanntmachung des Hinweises auf die Patenterteilung: **13.08.1997**
(21) Anmeldenummer: 94250178.4
(22) Anmeldetag: 07.07.1994
(51) Int. Cl.: C07J 41/00, A61K 31/565

(54) **Neue 11-Benzaldoximestradien-Derivate, Verfahren zu ihrer Herstellung und diese Verbindungen enthaltende Arzneimittel**
11-Benzaldoximeestradiene-derivates, a process for their preparation and pharmaceutical compositions containing them
4 Dérivés 11-benzaldoximes de estradiene, un procédé pour leur préparation et les compositions pharmaceutiques les contenant

(30) Priorität: 20.09.1993 DE 4332283
(43) Veröffentlichungstag der Anmeldung: 19.04.1995
(73) Patentinhaber: JENAPHARM GmbH, D-07745 Jena (DE)
(72) Erfinder: Schubert, Gerd, Dr., D-07743 Jena (DE); Kaufmann, Günther, Dr., D-07743 Jena (DE); Sobeck, Lothar, Dr., D-07743 Jena (DE); Oettel, Michael, Prof. Dr., D-07743 Jena (DE); Elger, Walter, Dr., D-14195 Berlin (DE); Kurischko, Anatoli, Dr., D-07745 Berlin (DE)
(74) Vertreter: Wablat, Wolfgang, Dr.Dr.

(56) Entgegenhaltungen:
- EP-A- 0 190 759
- EP-A- 0 245 170
- EP-A- 0 411 733
- EP-A- 0 648 779
- DD-A- 287 510
- DD-A- 289 539

## Beschreibung

Die Erfindung betrifft neue 11-Benzaldoxim-estradien-Derivate, Verfahren zu ihrer Herstellung und diese bindungen enthaltende Arzneimittel.

11β-substituierte Phenyl-estratriene sind bereits bekannt. Die Herstellung von 11β-Aryl-17α-propinyl-estra-4,9-dienen ist z. B. in der Patentschrift EP 057 115, die Umsetzung von 11β-(4-Formylphenyl)-estra-4,9-dien-3-onen mit Hydroxylaminen in der Patentschrift DE 3 504 421 bereits beschrieben. In dem angegebenen Verfahren wird sowohl die 11β-Formylphenylgruppe als auch die 3-Ketogruppe oximiert. Außerdem entstehen am C-3 syn-und anti-Isomere. Über die Wirkung der beschriebenen Verbindungen ist bisher nichts bekannt.

Progesteron wird während des Zyklus und in der Gravidität in großen Mengen vom Ovar und der Plazenta sezerniert. Seine regulatorische Bedeutung ist vermutlich nicht in allen Aspekten geklärt.

Gesichert ist, daß Progesteron im Zusammenwirken mit Oestrogenen die zyklischen Veränderungen der Uterusschleimhaut im Menstruationszyklus und der Gravidität bewirkt. Unter dem Einfluß erhöhter Progesteronspiegel nach der Ovulation wird die Uterusschleimhaut in einen Zustand überführt, der die Einnistung eines Embryos (Blastozyste) zuläßt. Die Erhaltung der Gewebe, in denen sich der wachsende Embryo ausbreitet, ist ebenfalls progesteronabhängig.

In der Gravidität findet eine dramatische Veränderung der muskulären Funktion des Uterus statt. Der gravide Uterusmuskel reagiert stark abgeschwächt oder gar nicht auf hormonale und mechanische Reize, die außerhalb der Gravidität Wehen auslösen. Es kann kein Zweifel daran bestehen, daß hierbei Progesteron eine Schlüsselrolle spielt, obwohl in manchen Phasen der Gravidität, z. B. unmittelbar vor der Geburt, eine hohe Reaktionsbereitschaft bei extrem hohen Blutspiegeln von Progesteron besteht.

Andere typische Vorgänge der Schwangerschaft sind ebenfalls Ausdruck der sehr hohen Progesteronspiegel. Der Aufbau der Brustdrüsen und der feste Verschluß des Muttermundes bis in die Nähe des Geburtstermins sind hierfür Beispiele.

Progesteron ist in subtiler Weise an der Steuerung von Ovulationsvorgängen beteiligt. Es ist bekannt, daß Progesteron in hohen Dosierungen antiovulatorische Eigenschaften besitzt. Diese resultieren aus einer Hemmung der hypophysären Gonadotropinsekretion, die Voraussetzung für die Reifung eines Follikels und dessen Ovulation ist. Andererseits ist erkennbar, daß die vergleichsweise geringe Progesteronsekretion des reifenden Follikels eine aktive Rolle für die Vorbereitung und Auslösung der Ovulation spielt. Hierbei spielen hypophysäre Mechanismen (zeitlich begrenzter sog. positiver feed back des Progesterons auf Gonadotropinsekretion) erkennbar eine wichtige Rolle (Loutradie, D.; Human Reproduction 6, 1991, 1238-1240).

Weniger gut analysiert sind Funktionen des Progesterons im reifenden Follikel und Gelbkörper selbst, an deren Existenz kein Zweifel besteht. Letztlich sind auch hier stimulierende und hemmende Effekte auf endokrine Follikel- und Gelbkörperfunktion anzunehmen.

Eine bedeutende Rolle des Progesterons und der gesteronrezeptoren ist auch in pathophysiologischen Prozessen anzunehmen. Progesteronrezeptoren sind in Endometrioseherden, aber auch in Tumoren des Uterus, der Mamma und des ZNS (Meningeome) nachgewiesen. Die Rolle dieser Rezeptoren für das Wachstumsverhalten dieser pathologisch relevanten Gewebe ist nicht notwendigerweise an das Vorliegen von Progesteronspiegeln im Blut gebunden. Es ist erweisen, daß Substanzen, die als gesteronantagonisten charakterisiert sind, RU 486 = Mifepriston (EP-0 057 115) und ZK 98299 = Onapriston (DE-OS-35 04 421) in diesen Geweben auch dann tiefgreifende Funktionsänderungen auslösen, wenn vernachlässigbar geringe Progesteronspiegel im Blut vorliegen. Es erscheint möglich, daß hierbei Änderungen von Transskriptionswirkungen des nicht von Progesteron besetzten gesteronrezeptors durch die Antagonisten eine wichtige Rolle spielen (Chwalisz, K. et al., Endocrinology, 129, 317-322, 1991).

Die Wirkungen von Progesteron in Geweben der Genitalorgane und anderen Geweben erfolgen durch Interaktion mit dem Progesteronrezeptor. In der Zelle bindet Progesteron mit hoher Affinität an seinen Rezeptor. Hierdurch werden Veränderungen des Rezeptorproteins bewirkt: Konformationsveränderungen, Dimerisierung von 2 Rezeptoreinheiten zu einem Komplex, Entblößung der DNA-Bindungsstelle des Rezeptors durch Abdissoziierung eines Proteins (HSP 90), Bindung an Hormon-responsible Elemente der DNA. Letztlich wird die Transskription bestimmter Gene reguliert. (Gronemeyer, H. et al., J. Steroid Biochem. Molec. Biol. 41, 3-8, 1992)

Die Wirkung von Progesteron oder von Progesteronantagonisten ist nicht nur von ihrer Konzentration im Blut abhängig. Die Konzentration von Rezeptoren in der Zelle ist ebenfalls stark reguliert. Oestrogene stimulieren die Synthese von Progesteronrezeptoren in den meisten Geweben. Progesteron hemmt die Synthese von Oestrogenrezeptoren und diejenige seines eigenen Rezeptors. Vermutlich sind es diese und andere interaktive Beziehungen zwischen Oestrogenen und Gestagenen, die erklären können, warum Gestagene und Antigestagene oestrogenabhängige Vorgänge beeinflussen können ohne daß sie vom Oestrogenrezeptor gebunden werden. Diese Beziehungen sind naturgemäß von großer Bedeutung für die therapeutische Anwendung von Antigestagenen. Diese Substanzen erscheinen geeignet, gezielt in Reproduktionsvorgänge der Frau einzugreifen, z. B. postovulatorisch, um die Nidation zu verhindern, in der späteren Gravidität, um Reaktionsbereitschaft des Uterus für Prostaglandine und Oxytocin zu erhöhen oder um eine Eröffnung und Erweichung ("Reifung") der Cervix zu erreichen.

Antigestagene hemmen bei verschiedenen subhumanen Primatenspezies die Ovulation. Der Mechanismus dieser Wirkung ist nicht eindeutig geklärt. Diskutiert werden neben einer Hemmung der Gonadotropinsekretion auch ovarielle Mechanismen durch Störung para- und autokriner Funktionen des Progesteron im Ovar.

Antigestagene haben die Fähigkeit, die Effekte von Oestrogenen zu modulieren oder abzuschwächen, obwohl sie selbst überwiegend keine Affinität zum Oestrogenrezeptor auf cytoplasmatischer Ebene besitzen und obwohl sie einen Anstieg der Oestrogenrezeptorkonzentration bewirken können. Von entsprechenden Effekten kann in Endometrioseherden bzw. in Tumorgeweben, das mit Oestrogen- und Progesteronrezeptoren ausgestattet ist, eine günstige Beeinflussung von Krankheitszuständen erwartet werden. Besondere Vorteile für die günstige Beeinflussung von Krankheitszuständen wie Endometriose könnten dann gegeben sein, wenn zu den Hemmeffekten eines Antigestagens durch die Wirkung im Gewebe eine Hemmung der Ovulation treten würde. Mit der Hemmung der Ovulation würde auch ein Teil der ovariellen Hormonproduktion und damit der auf diesen Anteil entfallende Stimulationseffekt auf das pathologisch veränderte Gewebe entfallen. Es wäre wünschenswert, bei Vorliegen einer schweren Endometriose die Ovulation zu hemmen und die sonst ständig im Umbau befindlichen Gewebe des Genitaltraktes reversibel in einen ruhenden Zustand zu versetzen.

Auch für die Kontrazeption wird ein Verfahren diskutiert, bei dem eine Antigestagenbehandlung die Ovulation unterdrückt, und durch eine anschließende Gestagenbehandlung die sekretorische Transformation des Endometriums induziert wird, so daß aus Behandlungstagen mit Antigestagenen und Gestagenen und behandlungsfreien Tagen ein ca. 28-tägiger Zyklus mit regelmäßigen Entzugsblutungen resultiert (Baulieu, E.E., Advances in Contraception 7, 345-51, 1991).

Antigestagene können unterschiedliche hormonale und antihormonale Eigenschaften besitzen. Von besonderer therapeutischer Relevanz sind hierbei antiglucocorticoide Eigenschaften. Diese sind für therapeutische Anwendungen, bei denen die Hemmung der Progesteronrezeptoren im Vordergrund der Therapie steht, nachteilig, da sie bei den therapeutisch erforderlichen Dosierungen unerwünschte Nebenwirkungen verursachen, die Applikation einer therapeutisch sinnvollen Dosis verhindern, oder zum Abbruch der Behandlung führen können. Die teilweise oder vollständige Reduktion antiglucocorticoider Eigenschaften ist eine wichtige Voraussetzung für die Therapie mit Antigestagenen, insbesondere für diejenigen Indikationen, die eine über Wochen oder Monate dauernde Behandlung erfordern.

Aufgabe der vorliegenden Erfindung ist es, neue 11β-Benzaldoximestra-4,9-dien-Derivate der allgemeinen Formel I 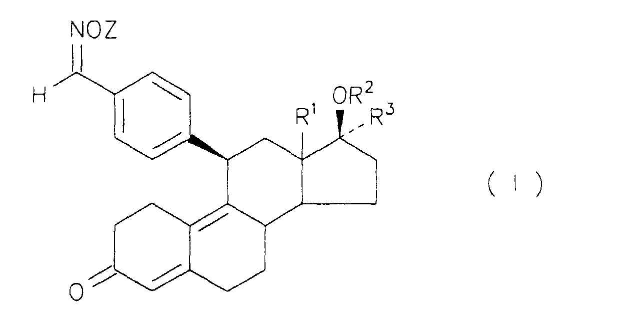 und ihre pharmazeutisch annehmbaren Salze sowie ein Verfahren zu ihrer Herstellung zur Verfügung zu stellen. Eine weitere Aufgabe ist es, Arzneimittel, die eine Verbindung der allgemeinen Formel I oder deren pharmazeutisch annehmbares Salz erhalten, zur Verfügung zu stellen.

In der allgemeinen Formel I, 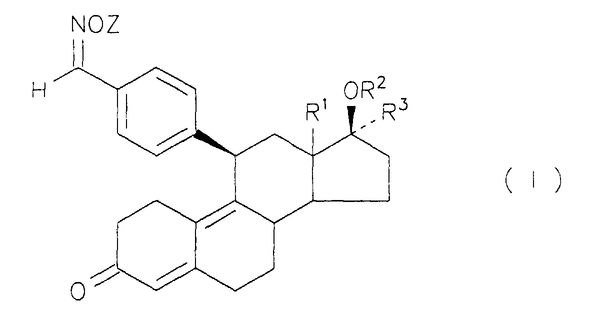 ist R¹ ein Wasserstoffatom oder ein Alkylrest mit 1-6 Kohlenstoffatomen,
steht R² für ein Wasserstoffatom, eine Alkyl-, Aryl-, Aralkyl- oder Alkylarylgruppe mit 1-10 Kohlenstoffatomen, einen Acylrest mit 1-10 Kohlenstoffatomen oder
einen Rest -CONHR⁴ oder -COOR⁴,
wobei R⁴ ein Wasserstoffatom, einen Alkyl-, Aryl-, Aralkyl- oder Alkylarylrest mit 1-10 Kohlenstoffatomen bedeutet,
bedeutet R³ ein Wasserstoffatom, eine Alkyl-, Aryl-, Aralkyl- oder Alkylarylgruppe mit 1 - 10 Kohlenstoffatomen,
einen Rest -(CH₂)ₙ-CH₂X,
wobei n = 0,1 oder 2 ist, X für ein Fluor-, Chlor-, Brom- oder Iodatom, für eine Cyano-, Azido- oder Rhodanogruppe, für einen Rest OR⁵ oder SR⁵ steht,
wobei R⁵ ein Wasserstoffatom, ein Alkyl-, Aryl-Aralkyl- oder Alkylarylrest mit 1-10 Kohlenstoffatomen oder ein Acylrest mit 1-10 Kohlenstoffatomen ist,
einen Rest OR⁵,
wobei R⁵ die oben angegebene Bedeutung hat,
einen Rest -(CH₂)ₒ-CH=CH(CH₂)ₚ-R⁶,
wobei o = 0, 1, 2 oder 3 und p = 0, 1 oder 2 ist und R⁶ für ein Wasserstoffatom, eine Alkyl-, Aryl-, Aralkyl- oder Alkylarylgruppe mit 1-10 Kohlenstoffatomen, eine Hydroxylgruppe, eine Alkoxygruppe oder Acyloxygruppe mit 1 - 10 Kohlenstoffatomen steht,
einen Rest -(CH₂)_{q}C≡CR⁷,
wobei q = 0, 1 oder 2 und R⁷ ein Wasserstoffatom, ein Fluor-, Chlor-, Brom- oder Iodatom,
ein Alkyl-, Aryl-, Aralkyl- oder Alkylarylrest mit 1-10 Kohlenstoffatomen oder ein Acylrest mit 1-10 Kohlenstoffatomen ist,
oder für den Fall, daß Z -H, R¹-CH₃, und R²-H oder -CH₃ ist, einen Rest -C≡C-CH₂OH,
steht Z für ein Wasserstoffatom, einen Alkyl-, Aryl-, Aralkyl- oder Alkylarylrest mit 1-10 Kohlenstoffatomen, einen Acylrest mit 1-10 Kohlenstoffatomen, einen Rest -CONHR⁴ oder -COOR⁴,
wobei R⁴ ein Wasserstoffatom, einen Alkyl-, Aryl-, Aralkyl- oder Alkylarylrest mit 1-10 Kohlenstoffatomen bedeutet,
oder für ein Alkali- oder Erdalkalimetallatom,
mit Ausnahme der Verbindungen, bei denen, R¹-CH₃, R²-CH₃ und R³-CH₂-O-CH₃ ist und Z für -CO-CH₃,-CO-O-C₂H₅, -CO-NH-Phenyl, -CO-NH-C₂H₅, -CO-C₂H₅ oder -CO-Phenyl steht.

Bevorzugt sind Verbindungen, in denen R¹ eine Methyl- oder Ethylgruppe ist,
R² für ein Wasserstoffatom, eine Alkylgruppe mit 1-10 Kohlenstoffatomen, einen Acylrest mit 1-10 Kohlenstoffatomen oder
einen Rest -CONHR⁴ oder -COOR⁴ steht,
wobei R⁴ ein Wasserstoffatom, einen Alkyl- oder Arylrest mit 1-10 Kohlenstoffatomen bedeutet,
R³ ein Wasserstoffatom, eine Alkyl-, Aryl-, Aralkyl- oder Alkylarylgruppe mit 1-10 Kohlenstoffatomen,
R³ einen Rest (CH₂)ₙ-CH₂X,
wobei n = 0,1 oder 2 ist, X für ein Fluor-, Chlor-, Brom- oder Iodatom, für eine Cyano-, Azido oder Rhodanogruppe, für einen Rest OR⁵ oder SR⁵ steht,
wobei R⁵ ein Alkylrest mit 1-6 Kohlenstoffatomen oder ein Acylrest mit 1-6 Kohlenstoffatomen ist,
einen Rest OR⁵,
wobei R⁵ ein Wasserstoffatom, ein Alkylrest mit 1-10 Kohlenstoffatomen oder ein Acylrest mit 1-10 Kohlenstoffatomen ist,
einen Rest -(CH₂)ₒ-CH=CH(CH₂)ₚ-R⁶,
wobei o = 0, 1, 2 oder 3 und p = 0,1 oder 2 ist und R⁶ für ein Wasserstoffatom, eine Alkylgruppe mit 1-10 Kohlenstoffatomen, eine Hydroxylgruppe, eine Alkoxygruppe oder Acyloxygruppe mit 1 - 10 Kohlenstoffatomen steht,
oder einen Rest-(CH₂)_{q}C≡CR⁷ darstellt,
wobei q = 0, 1 oder 2 und R⁷ ein Wasserstoffatom, ein Fluor-, Chlor-, Brom- oder Iodatom, ein Alkylrest mit 1-10 Kohlenstoffatomen oder ein Acylrest mit 1-10 Kohlenstoffatomen ist,
und Z für ein Wasserstoffatom, einen Alkylrest mit 1-10 Kohlenstoffatomen, einen Acylrest mit 1-10 Kohlenstoffatomen, einen
Rest -CONHR⁴ oder -COOR⁴,
wobei R⁴ ein Wasserstoffatom oder einen Alkylrest mit 1-10 Kohlenstoffatomen bedeutet.
oder für ein Alkali- oder Erdalkalimetallatom steht.

Besonders bevorzugt sind Verbindungen, in denen R² für eine Alkylgruppe mit 1-6 Kohlenstoffatomen, einen Acylrest mit 1-6 Kohlenstoffatomen oder
einen Rest -CONHR⁴ oder -COOR⁴ steht,
wobei R⁴ ein Wasserstoffatom, einen Alkyl- oder Arylrest mit 1-6 Kohlenstoffatomen bedeutet,
R³ ein Wasserstoffatom oder eine Alkylgruppe mit 1-6 Kohlenstoffatomen,
einen Rest -(CH₂)ₙ-CH₂X
wobei n = 0,1 oder 2 ist, X für ein Fluor-, Chlor-, Brom- oder Iodatom, für eine Cyano-, Azido oder Rhodanogruppe, für einen Rest OR⁵ oder SR⁵ steht,
wobei R⁵ ein Alkylrest mit 1-6 Kohlenstoffatomen oder ein Acylrest mit 1-6 Kohlenstoffatomen ist,
einen Rest OR⁵
wobei R⁵ ein Alkylrest mit 1-6 Kohlenstoffatomen oder ein Acylrest mit 1-6 Kohlenstoffatomen ist,
einen Rest -(CH₂)ₒ-CH=CH(CH₂)ₚ-R⁶,
wobei o = 0, 1, 2 oder 3 und p = 0,1 oder 2 ist und R⁶ für eine Alkylgruppe mit 1-6 Kohlenstoffatomen, eine Alkoxygruppe oder Acyloxygruppe mit 1-6 Kohlenstoffatomen steht,
oder einen Rest-(CH₂)_{q}C≡CR⁷ darstellt,
wobei q = 0, 1 oder 2 und R⁷ ein Alkylrest mit 1-6 Kohlenstoffatomen oder ein Acylrest mit 1-6 Kohlenstoffatomen ist,
und Z für einen Alkylrest mit 1-6 Kohlenstoffatomen, einen Acylrest mit 1-6 Kohlenstoffatomen, einen Rest -CONHR⁴ oder -COOR⁴ steht,
wobei R⁴ ein Wasserstoffatom, einen Alkyl- oder Arylrest mit 1-6 Kohlenstoffatomen bedeutet.

Am meisten bevorzugt sind:
11β-[4-(Hydroximinomethyl)phenyl]-17β-hydroxy-17α-methoxymethyl-estra-4,9-dien-3-on,
11β-[4-(Hydroximinomethyl)phenyl]-17β-hydroxy-17α-ethoxymethyl-estra-4,9-dien-3-on,
11β-[4-(Hydroximinomethyl)phenyl]-17β-hydroxy-17α-n-propoxymethyl-estra-4,9-dien-3-on,
11β-[4-(Hydroximinomethyl)phenyl]-17β-hydroxy-17α-i-propoxymethyl-estra-4,9-dien-3-on,
11β-[4-(Hydroximinomethyl)phenyl]-17β-methoxy-17α-methoxymethyl-estra-4,9-dien-3-on,
11β-[4-(Hydroximinomethyl)phenyl]-17β-methoxy-17α-ethoxymethyl-estra-4,9-dien-3-on,
11β-[4-(Hydroximinomethyl)phenyl]-17β-hydroxy-17α-(3-hydroxyprop-1-in-yl)-estra-4,9-dien-3-on,
11β-[4-(Hydroximinomethyl)phenyl]-17β-methoxy-17α-(3-hydroxyprop-1-in-yl)-estra-4,9-dien-3-on,
11β-[4-(Hydroximinomethyl)phenyl]-17β-hydroxy-17α-Z-(3-hydroxypropenyl)-estra-4,9-dien-3-on,
11β-[4-(Hydroximinomethyl)phenyl]-17β-methoxy-17α-Z-(3-hydroxypropenyl)-estra-4,9-dien-3-on,
17α-Chlormethyl-11β-[4-(hydroximinomethyl)phenyl]-17β-hydroxy-estra-4,9-dien-3-on,
17α-Chlormethyl-11β-[4-(hydroximinomethyl)phenyl]-17β-methoxy-estra-4,9-dien-3-on,
17α-Cyanomethyl-11β-[4-(hydroximinomethyl)phenyl]-17β-hydroxy-estra-4,9-dien-3-on,
17α-Cyanomethyl-11β-[4-(hydroximinomethyl)phenyl]-17β-methoxy-estra-4,9-dien-3-on,
17α-Azidomethyl-11β-[4-(hydroximinomethyl)phenyl]-17β-methoxy-estra-4,9-dien-3-on,
11β-[4-(Hydroximinomethyl)phenyl]-17β-methoxy-17α-methylthiomethyl-estra-4,9-dien-3-on,
11β-[4-(Methyloximinomethyl)phenyl]-17β-methoxy-17α-methoxymethyl-estra-4,9-dien-3-on,
11β-[4-(Methyloximinomethyl)phenyl]-17β-hydroxy-17α-methoxymethyl-estra-4,9-dien-3-on,
11β-[4-(Hydroximinomethyl)phenyl]-17β-ethoxy-17α-ethoxymethyl-estra-4,9-dien-3-on.

Die Erfindung betrifft ferner ein Verfahren zur Herstellung der Verbindungen nach der allgemeinen Formel I und deren pharmazeutisch annehmbaren Salze, das dadurch gekennzeichnet ist, daß man eine Verbindung der allgemeinen Formel II, 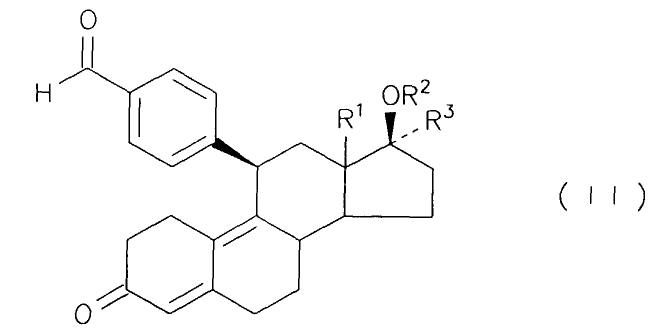 worin R¹, R² und R³ die vorstehend angegebene Bedeutung hat,
mit einer Verbindung der allgemeinen Formel IIa

NH₂-O-Y (IIa)

worin Y ein Wasserstoffatom, ein Alkylrest mit 1-10 Kohlenstoffatomen, ein Acylrest mit 1-10 Kohlenstoffatomen oder ein
Rest -CONHR⁴ oder -COOR⁴ ist,
wobei R⁴ ein Wasserstoffatom, einen Alkyl-, Aryl-, Aralkyl- oder Alkylarylrest mit 1-10 Kohlenstoffatomen bedeutet,
und wobei die Verbindung der allgemeinen Formel IIa gegebenfalls in Form einer solchen Verbindung vorliegt, oder aus der die Verbindung der allgemeinen Formel IIa unter den gewählten Reaktionsbedingungen freigesetzt wird,
umsetzt, die gegebenenfalls vorhandene Hydroximinogruppe zu Verbindungen der Formel I, in denen Z Kein H ist, verestert oder verethert und gegebenenfalls die erhaltene Verbindung in ein pharmazeutisch annehmbares Salz überführt.

Die Umsetzung der Verbindungen der allgemeinen Formel II und der allgemeinen Formel II a geschieht vorzugsweise, in dem man die Verbindungen in äquimolaren Mengen einsetzt.

Bei Wunsch nach Veresterung, Veretherung oder Urethan-Bildung von Verbindungen der allgemeinen Formel I, deren R²- oder Z-Substituent eine Hydroxylgruppe darstellt, kann die Veresterung in an sich bekannter Weise mit Acylierungsmitteln wie Säureanhydriden oder Säurechloriden in Gegenwart von Basen, vorzugsweise Pyridin, die Veretherung mit Methyljodid in Gegenwart von Basen, vorzugsweise Kalium-tert.-butanolat oder die Urethan-Bildung durch Reaktion mit Alkyl- oder Arylisocyanaten in inerten Lösungsmitteln, vorzugsweise Toluol oder durch Reaktion von Carbamoylchloriden in Gegenwart von Basen, vorzugsweise Triethylamin durchgeführt werden.

Die Herstellung der Ausgangsverbindung der allgemeinen Formel II erfolgt aus dem 5α,10α-Epoxid III 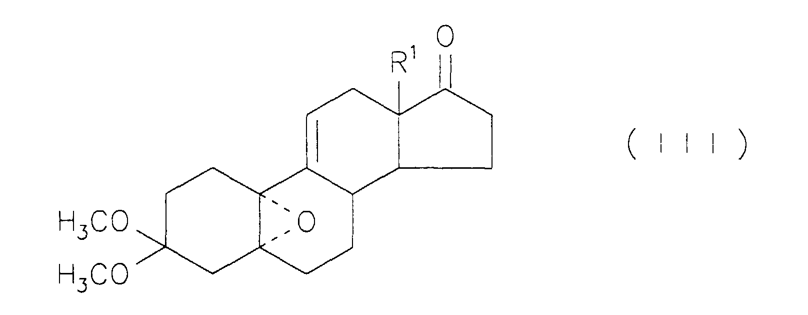 [vgl. z.B. Nédélec Bull. Soc. chim. France (1970), 2548].

Die Einführung des Phenylrestes in die 11β-Stellung unter Ausbildung einer Δ9(10), 5α-Hydroxy-Struktur IV wird durch Cu(I)-Salz katalysierte Grignard-Reaktion (Tetrahedron Letters 1979, 2051) mit einem p-Brombenzaldehydketal, vorzugsweise dem p-Brombenzaldehyddimethylketal bei Temperaturen zwischen 0°C und 30°C erreicht.

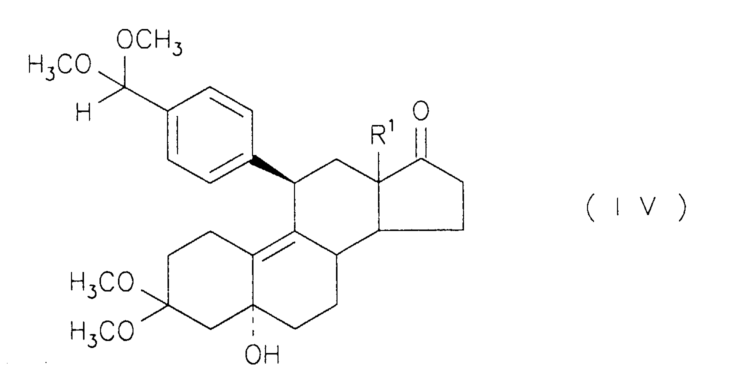

Die Einführung der Gruppierung -(CH₂)ₙCH₂X erfolgt nach dem an sich bekannten Weg über das Spiroepoxid V durch Umsetzung mit Trimethylsulfoniumjodid und Kalium-tert.-butanolat in Dimethylsulfoxid [Hübner et.al.; J. prakt. Chem. 314, 667 (1972); Arzneim. Forsch. 30, 401 (1973)] und 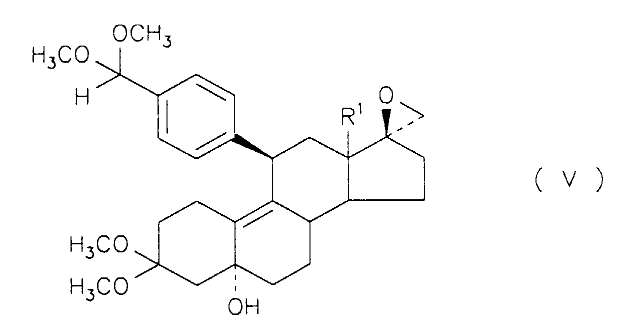 anschließende Ringöffnung mit Nucleophilen wie Halogeniden und Pseudohalogeniden, Alkoholaten und Mercaptiden [Ponsold et.al.; Z. Chem. 11, 106 (1971)]. Die dabei stehenden 17α-CH₂X-Verbindungen VI 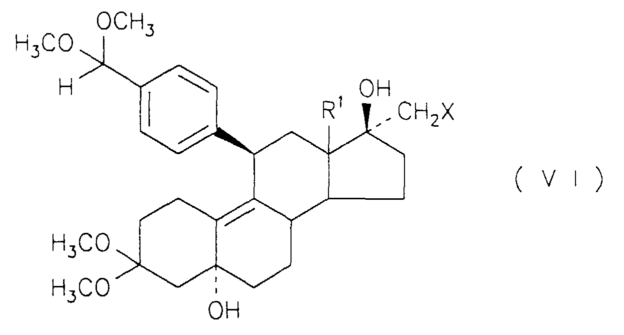 lassen sich entweder durch saure Hydrolyse, vorzugsweise mit p-Toluolsulfonsäure in Aceton (Teutsch et.al. DE 2801416) zu den Aldehyden der allgemeinen Formel II mit R² in der Bedeutung eines Wasserstoffatoms spalten oder nach verethern der freien Hydroxylgruppen mit Alkylhalogeniden in Gegenwart von Kalium-tert.-butanolat zunächst in die 5α,17β-Diether überführen (Kasch et.al. DD 290 893), die dann durch saure Hydrolyse, vorzugsweise mit p-Toluolsulfonsäure in Aceton, zu den Aldehyden der allgemeinen Formel II mit R² in der Bedeutung von Alkylresten, bevorzugt den Methylrest umgewandelt werden.

Die Einführung der Reste -(CH₂)ₒCH=CH(CH₂)ₚR⁶ erfolgt durch Umsetzung des Ketons IV mit Propin-1-ol-tetrahydropyranylether und Kalium-tert.-butanolat zu den 17α-(3-Hydroxy-1-propinyl)-17β-hydroxy-Verbindungen VII, 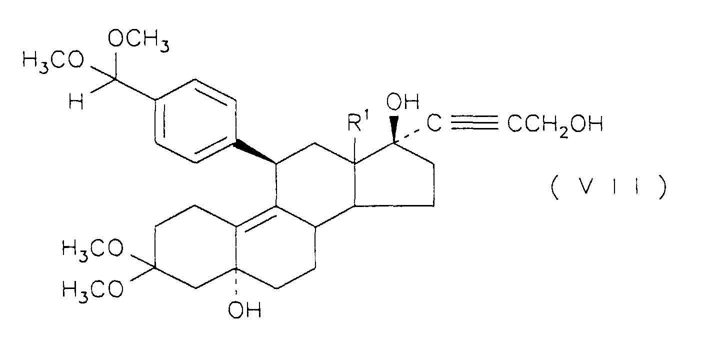 die entweder durch saure Hydrolyse unter den oben angegebenen Bedingungen zu den Aldehyden des Typ II mit R² in der Bedeutung eines Wasserstoffatoms hydrolisiert werden können oder nach Bildung der 5α,17β-Diether, nach den oben genannten Verfahren und nachfolgender saurer Hydrolyse zu den Aldehyden des Typ II mit R² in der Bedeutung eines Alkylrestes hydrolisiert werden oder aber in an sich bekannter Reaktion mit desaktivierten Katalysatoren wie 10 % Palladium auf Bariumsulfat in Gegenwart eines Amins zu den 17α-(3-Hydroxypropenyl)-17β-hydroxy-Verbindungen hydriert werden können, die nach saurer Hydrolyse ebenfalls in die Aldehyde des Typ II übergehen.

Die Einführung der Reste -(CH₂)_{q}C≡CR⁷ erfolgt in bekannter Weise durch Umsetzung des Ketons IV mit Acetylen, Propin oder höheren Homologen in Gegenwart von Alkalimetallen wie Lithium, Natrium oder Kalium in Verbindung mit einem Alkohol oder Ammoniak oder aber mit Butyllithium in Ethern wie Tetrahydrofuran. Die saure Hydrolyse dieser Verbindungen führt zu den 17α-C≡CR⁷ substituierten Aldehyden vom Typ II.

Gegebenenfalls wird die erhaltene erfindungsgemäße Verbindung der allgemeinen Formel I in ein Säureadditionssalz, vorzugsweise in ein Salz einer physiologisch verträglichen Säure überführt. Übliche physiologisch trägliche anorganische und organische Säuren sind spielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beispielweise in Fortschritte der neimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol, n-Propanol oder Isopropanol oder einem niederen Keton wie Aceton, Methyl-ethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können physiologisch verträgliche wäßrige Lösungen von Säureadditionssalzen der Verbindung der Formel I in einer wäßrigen Säurelösung hergestellt werden.

Die Säureadditionssalze der Verbindungen der allgemeinen Formel I können in an sich bekannter Weise, z. B. mit Alkalien oder Ionenaustauschern, in die freie Base überführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, rektalen, subcutanen, intravenösen oder intramuskulären Applikation, die neben üblichen Träger- und Verdünnungsmitteln eine Verbindung der allgemeinen Formel I oder deren Säureadditionssalz als Wirkstoff enthalten.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Die erfindungsgemäßen 11β-substituierten Benzaldoxim-estra-4,9-diene sind antigestagen wirkende Substanzen, die bei gleicher Aktivität wie RU 486 (Mifepristone) am Progesteron-Rezeptor (vgl. Tab. 1) bzw. bei überlegener Wirkung in vivo (vgl. Abb. 1 und Tab. 2) eine im Vergleich zu RU 486 deutlich reduzierte antiglucocorticoide Aktivität besitzen, nachgewiesen durch die verminderte Glucocorticoid-Rezeptorbindung (vgl. Tab. 1) sowie durch die ebenfalls um eine Zehnerpotenz geringere Enzyminduktionshemmung in Zellinien (vgl. Abb. 2 und 3).

**Tabelle 1**

| Rezeptorbindung von ausgewählten, unter Beispiel 1 bis 3 aufgeführten Substanzen | | | |
|---|---|---|---|
| Verbindung | relative molare Bindungsaffinität (RBA) [%] zum | | |
| nach Beispiel | Progesteron-Rez. | Glucocorticoid-Rez. | Estrogen-Rez. |
| | Progesteron = 100%) | (Dexamethason=100%) | (Estradiol=100%) |
| 1 (J867) | 302 | 78 | < 0,1 |
| 2 | 136 | 82 | < 0,1 |
| 3 | 90 | 32 | < 0,1 |
| z. Vergleich RU 486 (Mifepristone) | 506 | 685 | |
| ZK 98299 (Onapristone) | 22 | 39 | |

Ausgewählte erfindungsgemäße Antigestagene (J 687) führen bei Meerschweinchen im Zyklus zudem zu statistisch signifikant reduzierten Uterusgewichten bei Dosierungen, in denen RU 486 im Vergleich zu Kontrollen die Uterusgewichte erhöht.

Diese Kombination von Eigenschaften läßt von den erfindungsgemäßen Antigestagenen eine überlegene Hemmung von Progesteron, bei gleichzeitig reduzierter antiglucocorticoider Aktivität erwarten. Dieser Vorteil ist besonders im Hinblick auf Indikationen relevant, die wegen der Dauer der Anwendung eine besonders gute Verträglichkeit erfordern. Im Zyklus wird das Uterusgewicht entscheidend von zirkulierenden Oestrogenen bestimmt. Reduzierte Uterusgewichte sind Ausdruck einer Hemmung dieser Funktion der Oestrogene. Die festgestellte, RU 486 überlegene Hemmung der Uterusgewichte im Zyklus des Meerschweinchens, ist ein Hinweis auf (indirekte) antioestrogene Eigenschaften der erfindungsgemäßen Verbindungen. Von entsprechenden Effekten ist eine besonders günstige Beeinflussung von pathologisch veränderten Geweben zu erwarten, in denen Oestrogene Wachstumsimpulse vermitteln (Endometrioseherde, Myome, Mamma- und Genitalcarcinome, benigne Hypertrophie der Prostata).

**Tabelle 2**

| Frühabortive Wirkung von RU 486 und J 867 (Beispiel 1) bei der Ratte nach subkutaner Applikation vom 5. - 7. Graviditätstag (Applikation 0,2 ml/Tier/Tag in Benzylbenzoat/Rizinusöl [1 + 4 v/v]) | | | | |
|---|---|---|---|---|
| Gruppe Substanz | Dosis (mg/Tier/Tag) | komplette Graviditätshemmung⁺ | | ED 50 ⁺⁺ mg/Tier/Tag) |
| | | N* /N | % | |
| Vehikel | - | 0/13 | 0 | - |
| | | | | |
| RU 486 | 3,0 | 5/5 | 100 | |
| | 1,0 | 2/5 | 40 | 1,3 |
| | 0,3 | 0/5 | 0 | |
| | | | | |
| J 867 | 3,0 | 5/5 | 100 | |
| | 1,0 | 5/5 | 100 | 0,6 |
| | 0,3 | 0/5 | 0 | |
| | 0,1 | 0/5 | 0 | |

| | | | | |
|---|---|---|---|---|
| + - leere Uteri; | | | | |
| N - Zahl der besamten Weibchen; | | | | |
| N* - Zahl der nichtgraviden Weibchen; | | | | |
| ++ - graphische Bestimmung | | | | |

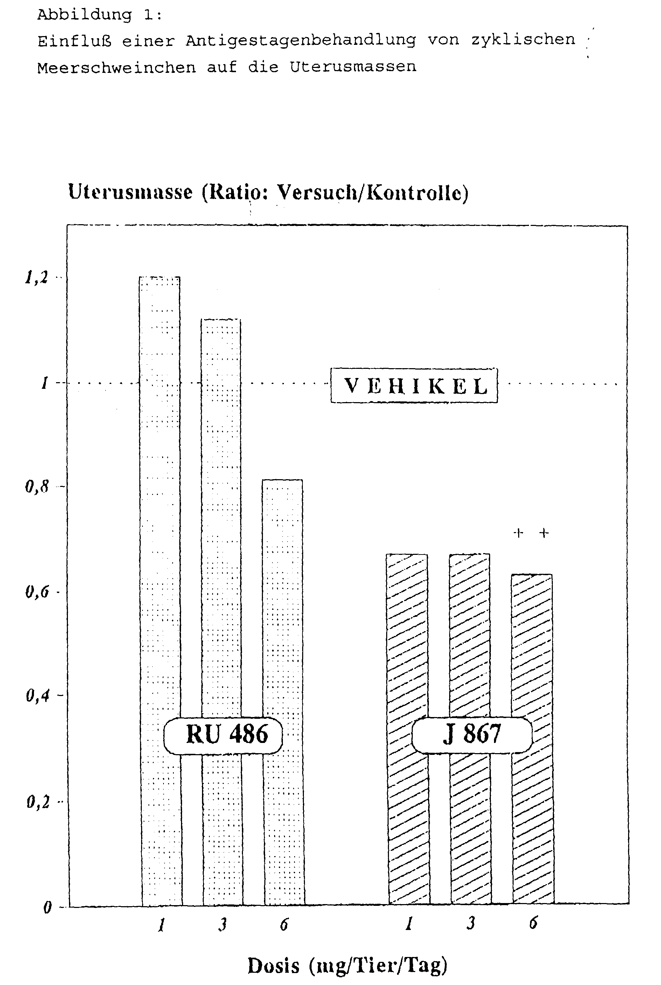

Die Abbildung 1 zeigt den Einfluß einer Antigestagenbehandlung von zyklischen Meerschweinchen auf die Uterusmassen im Vergleich von J 867 mit RU 486.

Hohe Dosierungen von RU 486 (6 mg/24 Std.) reduzieren die Uterusgewichte der behandelten Tiere. Niedrige Dosierung dieser Substanz führen dagegen zu einer leichten Erhöhung der Uterusgewichte. Alle geprüften Dosierungen von J 867 (1, 3 bzw. 6 mg/24 Std.) hemmten dagegen die Uterusgewichte statistisch signifikant.

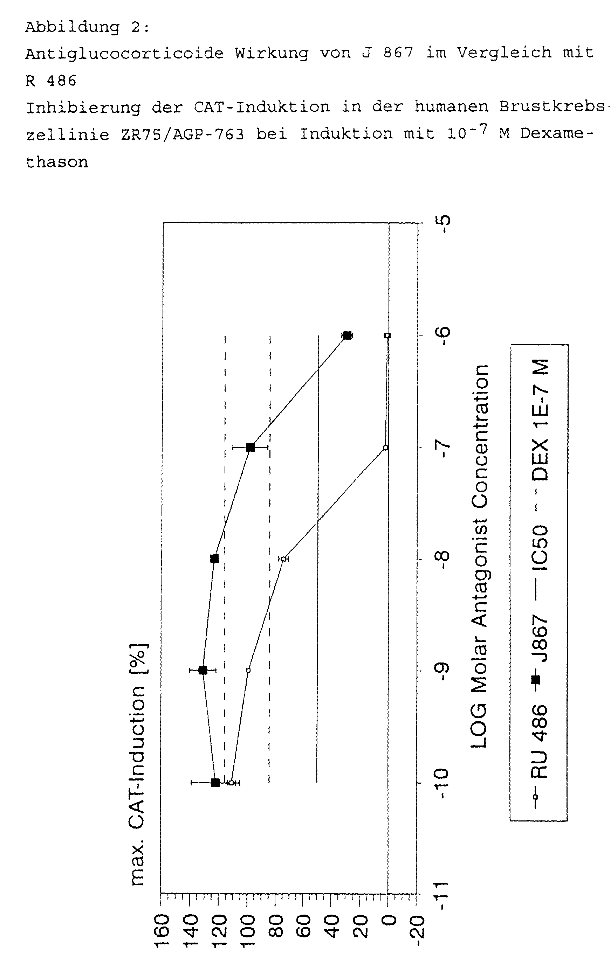

Die Abbildung 2 stellt die antiglucocorticoide Wirkung von J 867 in der humanen Brustzellinie ZR 75/AGP-763 im Vergleich zu RU 486 dar.

Dexamthason induziert in dieser Zellinie das Chloramphenicolacetyltransferase-Gen (CAT). Diese Induktion wird durch antiglucocorticoide Substanzen gehemmt. J 867 hemmt überraschenderweise in einem weiten Konzentrationsbereich die CAT weniger stark als RU 486 (Mifepristone).

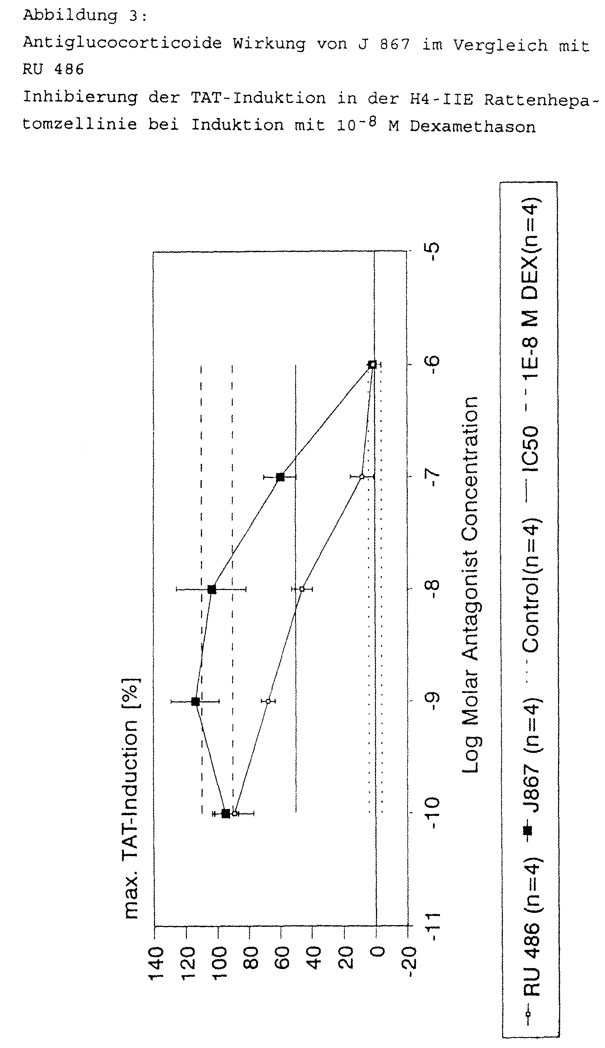

Abbildung 3 zeigt die Antiglucocorticoide Wirkung von J 867 im TAT-Modell in Rattenhepatomzellen im Vergleich mit RU 486.

In Hepatomzellen der Ratte stimuliert Dexamethason das Enzym Tyrosin-amino-transferase (TAT). Diese Wirkung wird durch anticlucocorticoide Aktivität gehemmt.
J 867 wirkt hiernach deutlich schwächer antiglucocorticoid als RU 486.

Die folgenden Beispiele erläutern die Erfindung

### Beispiele

### Beispiel 1

434 mg 11β-(4-Formylphenyl)-17β-methoxy-17α-methoxymethyl-estra-4,9-dien-3-on werden bei Raumtemperatur in 8 ml Pyridin gelöst und mit 65 mg Hydroxylaminhydrochlorid versetzt und bei 25°C gerührt. Nach 2 Stunden werden weitere 5 mg Hydroxylaminhydrochlorid zugegeben und die Lösung nach 15 Minuten mit Wasser verdünnt, mit 1 N wäßriger HCl versetzt und mit Chloroform extrahiert, die organische Phase mit verdünnter HCl und Wasser gewaschen, über Natriumsulfat und Kaliumcarbonat getrocknet und das Lösungsmittel unter vermindertem Druck verdampft. Man erhält 420 mg Rohprodukt. Nach Zugabe von Aceton fallen Kristalle aus, die abgesaugt und aus Isopropanol/CH₂Cl₂ umkristallisiert werden.

Es werden 305 mg 11β-[4-(Hydroximinomethyl)phenyl]-17β-methoxy-17α-methoxymethyl-estra-4,9-dien-3-on erhalten.
Schmelzpunkt: 118°C unter Zersetzung
α_{D} + 197° (CHCl₃)
IR-Spektrum in CHCl₃ (cm⁻¹): 3575, 3300 (OH); 1705 (C=NOH); 1649 (C=C-C=C-C=0); 1599 (Phenyl)

| | | |
|---|---|---|
| UV-Spektrum in MeOH: | λₘₐₓ = 264 nm | ε = 20 366 |
| | λₘₐₓ = 299 nm | ε = 20 228 |

¹H-NMR-Spektrum in CDCl₃ [δ, ppm]: 0,533 (s, 1H, H-18); 3,252 (s, 3H, 17β-OCH₃) ; 3,393 (s, 3H, 17α-CH₂OCH₃); 3,441-3,598 (m, 2H, ABX-System von CH₂OR) ; 4,381 (d, 1H, J=6,9 Hz, H-11α) ; 5,788 (s, 1H, H-4); 7,187-7,487 (m, 4H, AA'BB'-System der Aromatenprotonen); 8,05 (s, 1 H, OH); 8,097 (s, 1H, CH=NOH)
MS m/e: 449,25509 C₂₉H₃₅NO₄ M⁺

### Herstellung der Ausgangsverbindung:

### Stufe A

50 g 4-Brombenzaldehyd und 30 ml o-Ameisensäuretriethylester werden in 60 ml Methanol mit 0,8 mi Thionylchlorid bei Raumtemperatur 5 Stunden gerührt. Danach werden nochmals 0,2 ml Thionylchlorid zugegeben und nach 30 Minuten gießt man in wäßrige Bicarbonat-Lösung ein und extrahiert mit Chloroform, wäscht mit wäßriger Bicarbonat-Lösung und Wasser, trocknet über Natriumsulfat und engt unter vermindertem Druck ein und erhält 68 g 4-Brombenzaldehyddimethylketal als farbloses Öl.

Zu 2,3 g Magnesium in 20 ml wasserfreiem THF gibt man unter Argon als Schutzgas 0,2 ml Dibromethan und tropft nach Reaktionsbeginn 21,96 g 4-Brombenzaldehyddimethylketal in 70 ml wasserfreien THF derart zu, daß eine Temperatur von 40°C nicht überschritten wird. Nach vollständiger Zugabe wird 2 Stunden bei 30°C nachgerührt, anschließend auf -10°C abgekühlt und mit 511 mg CuCl versetzt. Es wird 15 Minuten bei -30°C weitergerührt und danach eine Lösung von 6 g 3,3-Dimethoxy-5α,10α-epoxyestr-9(11)-en-17-on in 30 ml wasserfreiem THF zugetropft. Man läßt auf Raumtemperatur erwärmen und zersetzt die Grignard-Lösung mit wäßriger Ammoniumchlorid-Lösung. Das Produkt wird durch Extraktion mit Essigester isoliert, die organische Phase neutral gewaschen und über Natriumsulfat getrocknet. Nach Abdestillation des Lösungsmittels unter vermindertem Druck werden 19,7 g Rohprodukt isoliert.

Chromatographie an 300 g Kieselgel und 20 g Aluminiumoxid mit einem Toluol/Essigester-Gradienten liefert 7,38 g 3,3-Dimethoxy-11β-[4-(dimethoxymethyl)phenyl)]-5a-hydroxyestr-9-en-17-on als gelben Schaum.

### Stufe B

7,38 g 3,3-Dimethoxy-11β-[4-(dimethoxymethyl)phenyl]-5α-hydroxy-estr-9-en-17-on werden in 85 ml Dimethylsulfoxid gelöst und unter Argon als Schutzgas mit 10,38 g Trimethylsulfoniumiodid und portionsweise mit 7,64 g Kalium- tert.-butanolat versetzt. Nach 1,5 Stunden wird auf 0-5°C abgekühlt und mit wäßriger Ammoniumchlorid-Lösung versetzt. Das klebrig ausfallende Produkt wird mit CH₂Cl₂ extrahiert, neutral gewaschen, über Natriumsulfat getrocknet und nach Verdampfung des Lösemittels als braunes Harz isoliert.

Ausbeute: 8,63 g 3,3-Dimethoxy-11β-[4-(dimethoxymethyl)phenyl]-17β-spiro-1',2'-oxiran-estr-9-en-5α-ol.

### Stufe C

8,63 g 3,3-Dimethoxy-11β-[4-(dimethoxymethyl)phenyl]-17β-spiro-1',2'-oxiran-estr-9-en-5α-ol werden in 20 ml Methanol gelöst und mit 20 ml 3N Natriummethylat-Lösung versetzt und 2 - 3 Stunden unter Rückfluß erhitzt. Unter vermindertem Druck wird das Lösungsmittel verdampft, der Rückstand in CH₂Cl₂ aufgenommen, neutral gewaschen, die Lösung über Natriumsulfat getrocknet und unter vermindertem Druck eingedampft.

Man erhält 8,74 g Rohprodukt als braunen Schaum. Chromatographie an 260 g Kieselgel und 90 g Aluminiumoxid mit einem Toluol/Essigester-Gradienten ergibt 1,92 g 3,3-Dimethoxy-11β-[4-(dimethoxymethyl)-phenyl]-17α-methoxymethyl-estr-9-en-5α,17β-diol.

### Stufe D

Zu 1,92 g 3,3-Dimethoxy-11β-[4-(dimethoxymethyl)phenyl]-17α-methoxymethyl-estr-9-en-5α,17β-diol in 120 ml Toluol werden unter Inertgas 8,65 g Kalium-*tert*.-butanolat gegeben. Man rührt die Suspension 5 Minuten bei Raumtemperatur und tropft 6,35 ml Methyliodid in 6 ml Toluol derart zu, daß die Temperatur nicht über 40°C steigt. Nach einer Stunde werden 20 ml Wasser und 20 ml Essigester zugegeben, die Phasen getrennt und die wäßrige Phase mit Essigester nachextrahiert. Die organische Phase wird neutral gewaschen, über Natriumsulfat getrocknet und unter vermindertem Druck verdampft.

Man erhält 1,55 g 3,3-Dimethoxy-11β-[4-(3,3-dimethoxymethyl)phenyl]-17α-methoxymethyl-estr-9-en-5α,17β-dimethylether als gelben Schaum.

### Stufe E

1,55 g 3,3-Dimethoxy-11β-[4-(3,3-dimethoxymethyl)phenyl]-17α-methoxymethyl-estr-9-en-5α,17β-dimethylether werden in 12,6 ml Aceton gelöst und mit 1,3 ml Wasser versetzt. Unter Schutzgas werden 158 mg 4-Toluolsufonsäure zugegeben. Man rührt 40 Minuten bei Raumtemperatur und saugt den Kristallbrei ab, wäscht mit Aceton und kristallisiert aus CH₂Cl₂/Aceton.

Ausbeute: 0,55 g farblose Kristalle.

Erneute Umkristallisation aus CH₂Cl₂/Aceton ergibt 440 mg 11β-(4-Formylphenyl)-17β-methoxy-17α-methoxymethyl-estra-4,9-dien-3-on.
Schmelzpunkt: 233 - 240°C
α_{D}= +189° (CHCl₃)
IR-Spektrum in CHCl₃ (cm⁻¹) :1710 (CHO); 1660 (C=C-C=C=0); 1610 (Phenyl)

| | | |
|---|---|---|
| UV-Spektrum in MeOH: | λₘₐₓ = 262 nm | ε = 10775 |
| | λₘₐₓ = 299 nm | ε = 13999 |

¹H-NMR-Spektrum in CDCl₃ [δ, ppm]: 0,48 (s, 3H, H-18); 3,25 (s, 3H, 17β-OCH₃); 3,38 (s, 3H, 17α-CH₂OCH₃); 4,40 (d, 1H, J=7,2 Hz; H-11α); 5,78(s, 1H, H-4); 7,28-7,93 (m, 4H, AA'BB'-System der Aromatenprotonen); 9,95 (s, 1H, CHO).
MS m/e: 434,24771 C₂₈H₃₄O₄ M⁺

### Beispiel 2

217 mg 11β-(4-Formylphenyl)-17β-methoxy-17α-methoxymethyl-estra-4,9-dien-3-on werden in 4 ml Pyridin mit 40 mg Methoxyaminhydrochlorid bei Raumtemperatur gerührt. Nach einer Stunde werden weitere 5,2 mg Methoxyaminhydrochlorid zugegeben. Es werden je 10 ml Wasser und Essigester zugesetzt, die Phasen getrennt, die wässrige Phase nachextrahiert, die organische Phase mit 10 ml verdünnter HCl gewaschen und mit dest. Wasser neutral gewaschen, getrocknet und unter vermindertem Druck verdampft. Man erhält 241 mg Rohprodukt als Harz. Präparative Schichtchromatographie an Kieselgel 60 PF ₂₅₄₊₃₆₆ mit den Laufmittelgemisch Toluol /Aceton (4:1) ergibt 188 mg Produkt als Schaum.

Umkristallisation aus Aceton/Hexan liefert 11β-[4-(Methoximinomethyl)phenyl]-17β-methoxy-17α-methoxymethyl-estra-4,9-dien-3-on als farblose Blättchen.
Schmelzpunkt: 83 - 89°C
α_{D}= +197° (CHCl₃)
IR-Spektrum in CHCl₃ [cm⁻¹]:
1700 (C=NOCH₃), 1649 (C=C-C=C-C=0), 1590 (Aromat)

| | | |
|---|---|---|
| UV-Spektrum in MeOH: | λₘₐₓ = 275 nm | ε = 23 098 |
| | λₘₐₓ = 300 nm | ε = 22 872 |

¹H-NMR-Spektrum in CDCl₃ [δ, ppm]: 0,529 (s, 3H, H-18); 3,247 (s, 3H, 17β-OCH₃); 3,408 (s, 3H, 17α-CH₂OCH₃); 3,39-3,598 (m, 2H, ABX-System, 17α-CH₂O-CH₃); 4,381 (d, 1H, J=7,5 Hz, H-11α); 5,773 (s, 1H, H-4); 7,173, 7,201, 7,463, 7,491 (m, 4H, AA'BB'-System der Aromatenprotonen); 8,023(s, 1H, CHPhenyl).
MS m/e: 463,26950 C₂₉H₃₇NO₄ M⁺

### Beispiel 3

244 mg 11β-(4-Formylphenyl)-17β-hydroxy-17α-chlormethylestra-4,9-dien-3-on werden in 4 ml Pyridin mit 32,2 mg Hydroxylaminhydrochlorid bei Raumtemperatur gerührt. Nach einer Stunde werden weitere 6,9 mg Hydroxylaminhydrochlorid zugegeben. Danach werden je 10 ml Wasser und Essigester zugesetzt, die Phasen getrennt, die wäßrige Phase nachextrahiert, die organische Phase mit 10 ml verdünnter HCl und mit dest. Wasser neutral gewaschen, getrocknet und unter vermindertem Druck verdampft.

Man erhält 183 mg Rohprodukt als gelbes Harz. Präparative Schichtchromatographie an Kieselgel 60 PF₂₅₄₊₃₆₆ mit dem Laufmittelgemisch Toluol/Aceton (4:1) ergibt 87,7 mg 17α-Chlormethyl-11β-[4-(hydroximinomethyl)phenyl]-17β-hydroxyestra-4,9-dien-3-on als Schaum.
α_{D} = + 185° (CHCl₃)

| | | |
|---|---|---|
| UV-Spektrum in MeOH: | λₘₐₓ = 264 nm | ε = 20 797 |
| | λₘₐₓ = 299 nm | ε = 20 439 |

¹H-NMR-Spektrum in CDCl₃ [δ, ppm]: 0,607 (s, 3H, H-18); 3,628; 3,664, 3,824, 3,861 (m, 2H, ABX-System, 17α-CH₂Cl); 4,428 (d, 1H, J=6,9 Hz, H-11α); 5,807 (s, 1H, H-4); 7,185, 7,212, 7,482, 7,509 (m, 4H, AA'BB'-System der Aromatenprotonen); 8,05 (s, 1H, OH), 8,104 (s, 1H, CHPhenyl).
MS m/e: 439,19070 C₂₆H₃₀ClNO₃ M⁺

### Herstellung der Ausgangsverbindung

### Stufe F

Zu 4,12 g 3,3-Dimethoxy-11β-[4-(dimethoxymethyl)phenyl]-17β-spiro-1', 2'-oxiran-estr-9-en-5α-ol (nach Beispiel 1 Stufe B hergestellt) in 84 ml Dimethylformamid werden bei 0°C 16,6 ml konz. HCl langsam zugetropft. Nach einer Stunde wird in 420 ml Wasser eingerührt, wobei ein flockiger Niederschlag entsteht. Mit wäßriger Bicarbonat-Lösung wird auf pH 6 gestellt, der Niederschlag abgesaugt und getrocknet.
Rohausbeute : 3,38 g ockerfarbene Kristalle, die durch Säulenchromatographie an 90 g Kieselgel 60 mit einem Toluol/Essigester-Gradienten gereinigt werden.

Ausbeute: 1,16 g 17α-Chlormethyl-11β-(4-formylphenyl)-17β-hydroxy-estra-4,9-dien-3-on als Kristalle.
Schmelzpunkt: 205 - 208°C (Aceton/Hexan)
α_{D} = +161° (CHCl₃)
IR-Spektrum in CHCl₃ [cm⁻¹]: 3600 (OH); 1695 (CHO); 1650 (C=C-C=C-C=C; 1590 (Phenyl)

| | | |
|---|---|---|
| UV-Spektrum in MeOH: | λₘₐₓ = 262 nm | ε = 19 993 |
| | λₘₐₓ = 297 nm | ε = 22 755 |

¹H-NMR-Spektrum in CDCl₃ [δ, ppm]: 0,583 (s, 3H, H-18); 3,30 (s, 1H, OH); 3,63-3,85 (m, 2H, ABX-System von CH₂Cl); 4,45 (d, 1H, J=7,0 Hz, H-11α); 5,809 (s, 1H, H-4); 7,355, 7,382, 7,799, 7,826 (m, 4 H, AA'BB'-System der Aromatenprotonen); 9,977 (s, 1H, CHO)
MS m/e: 424,18280 C₂₆H₂₉ClO₃ M⁺

### Beispiel 4

136 mg 11β-(4-Formylphenyl)-17β-hydroxy-17α-methoxymethyl-estra-4,9-dien-3-on werden bei Raumtemperatur in 2,2 ml Pyridin gelöst und mit 18 mg Hydroxylaminhydrochlorid versetzt und bei 25°C gerührt. Nach 1,5 Stunden werden weitere 4 mg Hydroxylaminhydrochlorid zugegeben und die Lösung nach 15 Minuten mit Wasser verdünnt, mit 1N wäßriger HCl versetzt und mit Chloroform extrahiert, die organische Phase mit verdünnter HCl und Wasser gewaschen, über Natriumsulfat und Kaliumcarbonat getrocknet und das Lösungsmittel unter vermindertem Druck verdampft.

Man erhält 146 mg Rohprodukt, das durch präparative Schichtchromatographie an Kieselgel 60 PF₂₅₄₊₃₆₆ mit dem Laufmittelgemisch Toluol/Aceton (4:1) gereinigt wird.

Man erhält 110 mg 11β-[4-(Hydroximinomethyl)phenyl]-17β-hydroxy-17α-methoxymethyl-estra-4,9-dien-3-on.

Schmelzpunkt: 104°C unter Zersetzung (Isopropanol)
α_{D} = + 195° (CHCl₃)

| | | |
|---|---|---|
| UV-Spektrum in MeOH: | λₘₐₓ = 263 nm | ε = 21 170 |
| | λₘₐₓ = 299 nm | ε = 20 188 |

¹H-NMR-Spektrum in CDCl₃ [δ, ppm]: 0,517 (s, 1H, H-18); 3,418 (s, 3H, 17α-CH₂OCH₃); 3,206, 3,237, 3,552, 3,582 (m, 2H, ABX-System von CH₂OCH₃); 4,384 (d, 1H, J=7,2 Hz, H-11α) 5,784 (s, 1H, H-4); 7,179, 7,206, 7,456, 7,483 (m, 4H, AA'BB'-System der Aromatenprotonen); 7,9 (s, 1H, OH); 8,088 (s, 1H, CH=NOH).
MS m/e: 435,24289 C₂₇H₃₃NO₄ M+

### Herstellung der Ausgangsverbindung

### Stufe G

860 mg 3,3-Dimethoxy-11β-[4-(dimethoxymethyl)phenyl]-17α-methoxymethyl-estr-9-en-5α,17β-diol (nach Beispiel 1 Stufe C hergestellt) werden in 80 ml Aceton gelöst. Nach Zugabe von 7,7 ml Wasser und 430 mg 4-Toluolsulfonsäure wird noch 1,5 Stunden am Rückfluß erhitzt unter vermindertem Druck eingeengt, der Rückstand in Chloroform aufgenommen, mit 8 ml verdünntem Ammoniak auf pH 8 gestellt und die Phasen getrennt. Die organische Phase wird neutral gewaschen, über Natriumsulfat getrocknet und unter vermindertem Druck verdampft.

Man erhält 590 mg 11β-(4-Formylphenyl)-17β-hydroxy-17α-methoxymethyl-estra-4,9-dien-3-on-1 als Rohprodukt, das aus Essigester umkristallisiert wird.
Schmelzpunkt: 195 - 205°C (Essigester)
α_{D}= + 209° (Chloroform)
IR-Spektrum in CHCl₃ [cm-1]: 3590 (OH); 1710 (CHO); 1660 (C=C-C=C-C=0); 1605 (Aromat)

| | | |
|---|---|---|
| UV-Spektrum in MeOH: | λₘₐₓ = 263 nm | ε = 20 683 |
| | λₘₐₓ = 298 nm | ε = 20 749 |

¹H-NMR-Spektrum in CDCl₃ [δ, ppm]: 0,509 (s, 3H, H-18); 2,666 (s, 1H, OH) 3,196, 3,226, 3,550, 3,580 (m, 211 ABX-System von CH₂OCH₃) ; 3,417 (s, 3H, OCH₃) ; 4,446 (d, 1H, J=6,9 Hz, H-11α); 5,797 (s, 1H, H-4); 7,360, 7,386, 7,786, 7,813 (m, 4H, AA'BB'-System der Aromatenprotonen); 9,970 (s, 1H, CH0)
MS m/e: 420,23300 C₂₇H₃₂O₄ M⁺

### Beispiel 5

480 mg 17α-Ethoxymethyl-11β-(4-formylphenyl)-17β-methoxyestra-4,9-dien-3-on werden bei Raumtemperatur in 6 ml Pyridin gelöst, mit 74 mg Hydroxylaminhydrochlorid versetzt und bei 25°C gerührt. Nach 30 Minuten werden weitere 8,5 mg Hydroxylaminhydrochlorid zugegeben und die Lösung nach 15 Minuten mit Wasser verdünnt, mit 1N wäßriger HCl versetzt und mit CH₂Cl₂ extrahiert, die organische Phase mit verdünnter HCl und Wasser gewaschen, über Natriumsulfat und Kaliumcarbonat getrocknet und das Lösungsmittel unter vermindertem Druck verdampft.

Man erhält 410 mg Rohprodukt, das nach präparativer Schichtchromatographie an Kieselgel 60 PF₂₅₄₊₃₆₆ 230 mg 17α-Ethoxymethyl-11β-[4-(hydroximinomethyl)phenyl]-17β-methoxy-estra-4,9-dien-3-on als gelben Schaum ergibt.
α_{D}= + 200° (CHCl₃)

| | | |
|---|---|---|
| UV-Spektrum in MeOH: | λₘₐₓ = 264 nm | ε = 20 366 |
| | λₘₐₓ = 299 nm | ε = 20 228 |

¹H-NMR-Spektrum in CDCl₃ [δ, ppm]: 0,533 (s, 1H, H-18); 1,267 (t, 3H, J=6,9 Hz, 17α-CH₂OCH₂CH₃), 3,252 (s, 3H, 17 β-OCH₃); 3,423 - 3,623 (m, 2H, ABX-System, 17α-CH₂OCH₂CH₃); 4,355 (d, 1H, J = 7,2 Hz, H-11α); 5,783 (s, 1H, H-4); 7,191, 7,219, 7,460, 7,488 (m, 4H, AA'BB'-System der Aromatenprotonen); 8,097 (s, 1H, CH=NOH
MS m/e: 463,27069 C₂₉H₃₇NO₄ M+

### Herstellung der Ausgangsverbindung

### Stufe H

5,33 g 3,3-Dimethoxy-11β-[4-(dimethoxymethyl)phenyl]-17β-spiro-1',2'-oxiran-estr-9-en-5α-ol (nach Beispiel 1 Stufe B hergestellt) werden in 5 ml Ethanol gelöst und mit 25 ml 1,5 N Natriumethylat-Lösung versetzt und eine Stunde unter Rückfluß erhitzt. Unter vermindertem Druck wird das Lösungsmittel verdampft, der Rückstand in CH₂Cl₂ aufgenommen, neutral gewaschen, die Lösung über Natriumsulfat getrocknet und unter vermindertem Druck eingedampft.

Man erhält 5,85 g Rohprodukt als braunen Schaum. Chromatographie an Kieselgel mit einem Toluol/EssigesterGradienten ergibt 1,14 g 3,3-Dimethoxy-11β-[4-(dimethoxymethyl)phenyl]-17α-ethoxymethyl-estr-9-en-5α,17β-diol.

### Stufe I

Zu 1,14 g 3,3-Dimethoxy-11β-[4-(dimethoxymethyl)phenyl]17α-ethoxymethyl-estr-9-en-5α,17β-diol in 70 ml Toluol werden unter Argon als Schutzgas 5,14 g Kalium-tert.- butanolat gegeben. Nach 15 Minuten tropft man eine Mischung aus 3,8 ml Methyliodid in 4 ml Toluol zu. Nach 2 Stunden wird durch Zugabe von 20 ml Wasser und 20 ml Essigester die Reaktion abgebrochen. Die organische Phase wird zweimal mit Wasser gewaschen, über Natriumsulfat getrocknet und unter vermindertem Druck verdampft.

Rohausbeute: 1,14 g 3,3-Dimethoxy-11β-[4-(3,3-dimethoxymethyl)phenyl]-17α-ethoxymethyl-17β-methoxy-estr-9-en-5α-ol.

### Stufe J

Zu 1,14 g 3,3-Dimethoxy-11β-[4-(dimethoxymethyl)phenyl]-17α-ethoxymethyl-17β-methoxy-estr-9-en-5α-ol in 10 ml Aceton werden unter Inertgas 1 ml Wasser und 116 mg 4-Toluolsulfonsäure zugegeben. Nach 30 Minuten wird mit Wasser verdünnt, mit Essigester zweimal extrahiert, die organische Lösung gewaschen und über Natriumsulfat getrocknet. Nach Verdampfung des Lösungsmittels verbleiben 900 mg 17α-Ethoxymethyl-11β-(4-formylphenyl)-17β-methoxyestra-4,9-dien-3-on als gelber Schaum. Chromatographie an Kieselgel 60 mit einem Toluol/Essigester-Gradienten ergibt 480 mg gelbe Kristalle.

### Beispiel 6

244 mg 11β-(4-Formylphenyl)-17β-hydroxy-17α-(3-hydroxyprop-1-in-yl)-estra-4,9-dien-3-on werden bei Raumtemperatur in 4,5 ml Pyridin gelöst und mit 35,5 mg Hydroxylaminhydrochlorid versetzt und bei 25°C gerührt. Nach 30 Minuten werden weitere 4,8 mg Hyroxylaminhydrochlorid zugegeben und die Lösung nach 15 Minuten mit Wasser verdünnt, in Essigester aufgenommen, mit 1 N wäßriger HCl ausgeschüttelt, die organische Phase mit Wasser gewaschen, über Natriumsulfat und Kaliumcarbonat getrocknet und das Lösungsmittel unter vermindertem Druck verdampft.

Man erhält 216 mg Rohprodukt, das nach präparativer Schichtchromatographie an Kieselgel 60 PF₂₅₄₊₃₆₆ mit dem Laufmittelgemisch Toluol/Aceton (4:1) 192 mg 11β-[4-(Hydroximinomethyl)phenyl]-17β-hydroxy-17α-(3-hydroxyprop-1-in-yl)-estra-4,9-dien-3-on als farblosen Schaum ergibt.
Schmelzpunkt 171 - 179°C (Ether)
α_{D} = +82° (Dioxan)

| | | |
|---|---|---|
| UV-Spektrum in MeOH: | λₘₐₓ = 264 nm | ε = 21 495 |
| | λₘₐₓ = 299 nm | ε = 20 236 |
| MS m/e: 445,22369 | C₂₈H₃₁NO₄ M⁺ | |

### Herstellung der Ausgangsverbindung:

### Stufe K

Zu 3 ml Prop-1-in-yl-3-hydroxytetrahydropyranylether in 27 ml wasserfreiem THF werden bei -5°C 12 ml 15 %iges n-Butyllithium in Hexan zugetropft. Nach 15 Minuten läßt man zu dieser Lösung 1,2 g 3,3-Dimethoxy-11β-[4-(dimethoxymethyl)phenyl]-5α-hydroxy-estr-9-en-17-on (nach Beispiel 1 Stufe B hergestellt) in 16 ml wasserfreiem THF zutropfen. Es wird 30 Minuten bei Raumtemperatur gerührt, die Reaktionslösung in 150 ml Eiswasser gegossen und mit Essigester extrahiert. Die organische Phase wird neutral gewaschen, über Natriumsulfat getrocknet und unter vermindertem Druck verdampft.

Man erhält 4,23 g braunes Öl, das durch Chromatographie an Kieselgel 60 mit einem Toluol/Essigester-Gradienten gereinigt wird. Man erhält 422 mg 3,3-Dimethoxy-11β-[4-(dimethoxymethyl)phenyl]-17α-(3-tetrahydropyranyloxyprop-1-in-yl)-estr-9-en-5α,17β-diol als Schaum.

### Stufe L

540 mg 3,3-Dimethoxy-11β-[4-(dimethoxymethyl)phenyl]-17α-(3-tetrahydropyranyloxy-prop-1-in-yl)-estr-9-en-5α,17β-diol werden in 40 ml Aceton mit 100 mg 4-Toluolsulfonsäure 2 Stunden bei Raumtemperatur gerührt. Danach wird auf 10 ml eingeengt, mit wäßriger Natriumbicarbonat-Lösung versetzt und mit Essigester extrahiert. Die organische Phase wird neutral gewaschen, über Natriumsulfat getrocknet und unter vermindertem Druck verdampft.

Rohausbeute: 330 mg. Die Reinigung durch präparative Schichtchromatographie an Kieselgel 60 PF₂₅₄₊₃₆₆ ergibt 310 mg 11β-(4-Formylphenyl)-17β-hydroxy-17α-(3-hydroxyprop-1-in-yl)-estra-4,9-dien-3-on. Umkristallisation aus Aceton liefert weiße Kristalle.
Schmelzpunkt: 225 - 231°C.
α_{D}=+ 59° (Chloroform)

| | | |
|---|---|---|
| UV-Spektrum in MeOH: | λₘₐₓ = 302 nm | ε = 23 608 |

¹H-NMR-Spektrum in CDCl₃ [δ, ppm]: 0,496 (s, 3H, H-18); 4,375 (s, 2H, C-CH₂OH); 4,497 (d, 1H, J=7,2 Hz, H-11α); 5,810 (s, 1H, H-4); 7,353, 7,380, 7,797, 7,824 (m, 4H, AA'BB'-System der Aromatenprotonen); 9,974 (s, 1H, CHO)
MS m/e: 430,21460 C₂₈H₃₀O₄ M⁺

### Beispiel 7

125 mg 17α-Ethoxymethyl-11β-(4-formylphenyl)-17β-hydroxyestra-4,9-dien-3-on werden bei Raumtemperatur in 2 ml Pyridin gelöst, mit 20,2 mg Hydroxylaminhydrochlorid versetzt und bei 25°C gerührt. Nach 50 Minuten wird die Lösung mit Wasser verdünnt, mit Essigester versetzt und die Phasen getrennt. Die organische Phase wird mit verdünnter HCl und Wasser gewaschen, über Natriumsulfat getrocknet und das Lösungsmittel unter vermindertem Druck verdampft.

Man erhält 127 mg Rohprodukt als hellgelben Schaum, der nach präparativer Schichtchromatographie an Kieselgel 60 PF₂₅₄₊₃₆₆ 62 mg 17α-Ethoxymethyl-11β-[4-(hydroximinomethyl)phenyl]-17β-hydroxy-estra-4,9-dien-3-on als farblosen Schaum ergibt.
α_{D} = + 226° (CHCl₃)

| | | |
|---|---|---|
| UV-Spektrum in MeOH: | λₘₐₓ = 265 nm | ε = 22 696 |
| | λₘₐₓ = 299 nm | ε = 21 960 |

¹H-NMR-Spektrum in CDCl₃ [δ,ppm]: 0,520 (s, 1H, H-18); 1,249 (t, 3H, J=7,2 Hz, 17α-CH₂OCH₂CH₃); 3,228-3,609 (m, 4H, 2x CH₂); 4,381 (d, 1H, J=7,2 Hz, H-11α) 5,781 (s, 1H, H-4); 7,181, 7,209, 7,459, 7,486 (m, 4H, AA'BB'-System der Aromatenprotonen); 8,098 (s, 1H, CH=NOH).
MS m/e: 449,25540 C₂₈H₃₅NO₄ M⁺

### Herstellung der Ausgangsverbindung

### Stufe M

340 mg 3,3-Dimethoxy-11β-[4-(dimethoxymethyl)phenyl]-17α-ethoxymethyl-estr-9-en-5α,17β-diol (nach Beispiel 5 Stufe H hergestellt) werden in 2,5 ml Aceton gelöst, mit 0,25 ml Wasser und 35 mg 4-Toluolsulfonsäure versetzt. Nach 1 Stunde wird mit 10 ml Wasser verdünnt und 10 ml Essigester zugesetzt. Die Phasen werden getrennt, die wäßrige Lösung zweimal nachextrahiert, die organische Lösung gewaschen und über Natriumsulfat getrocknet. Nach Verdampfung des Lösungsmittels verbleiben 300 mg gelbliche Kristalle.

Nach präparativer Schichtchromatographie an Kieselgel 60 PF₂₅₄₊₃₆₆ mit Toluol/Aceton (9:1) werden 200 mg 17α-Ethoxymethyl-11β-(4-formylphenyl)-17β-hydroxy-estra-4,9-dien-3-on als gelbliche Kristalle erhalten.
Schmelzpunkt: 144 - 150°C (Aceton/Hexan)
α_{D} = +171° (CHCl₃)

| | | |
|---|---|---|
| UV-Spektrum in MeOH: | λₘₐₓ = 263 nm | ε = 17 842 |
| | λₘₐₓ = 299 nm | ε = 20 083 |

¹H-NMR-Spektrum in CDCl₃ [δ,ppm]: 0,512 (s, 1H, H-18); 1,249 (t, 3H, ΣJ=4,6 Hz, 17α-CH₂OCH₂CH₃); 3,221 - 3,613 (m, 4H, 2 x CH₂); 4,447 (d, 1H, J=6,9 Hz, H-11α); 5,799 (s, 1H, H-4); 7,361, 7,388, 7,788, 7,816 (m, 4H, AA'BB'-System der Aromatenprotonen); 9,972 (s, 1H, CH=0).

### Beispiel 8

Messung der Rezeptor-Bindungsaffinität:

Die Rezeptorbindungsaffinität wurde bestimmt durch competitive Bindung eines spezifisch bindenden ³H-markierten Hormons (Tracer) und der zu testenden Verbindung an Rezeptoren im Cytosol aus tierischen Target-Organen. Dabei wurden Rezeptorsättigung und Reaktionsgleichgewicht angestrebt. Folgende Inkubationsbedingungen wurden gewählt:

Progesteron-Rezeptor: Uterus-Cytosol des Estradiol-geprimten Kaninchens, aufbewahrt bei -30°C, in TED-Puffer (20 mM Tris/HCl, pH 7,4; 1 mM Ethylendiamintetraacetat, 2 mM Dithiothreitol) mit 250 mM Saccharose. Tracer: ³H-ORG 2058, 5 nM; Referenzsubstanz: Progesteron.

Glucocorticoid-Rezeptor: Thymus-Cytosol der adrenalectomierten Ratte, Thymi aufbewahrt bei -30°C; Puffer: TED. Tracer: ³H-Dexamethason, 20 nM; Referenzsubstanz: Dexamethason.

Estrogen-Rezeptor: Uterus-Cytosol des unreifen Kaninchens, aufbewahrt bei -30°C in TED-Puffer mit 250 mM Saccharose. Tracer: ³H-Ethinylestradiol, 3 nM; Referenzsubstanz: 17β-Estradiol.

Nach einer Inkubationsdauer von 18 Stunden bei 0 - 4°C erfolgte die Trennung von gebundenem und freiem Steroid durch Einmischen von Aktivkohle/Dextran (1%/0,1%), Abzentrifugieren und Messung der gebundenen ³H-Aktivität im Überstand.

Aus Messungen in Konzentrationreihen wurden die IC₅₀ für die zu testende Verbindung und für die Referenzsubstanz ermittelt und als Quotient beider Werte (x 100%) die relative molare Bindungsaffinität.

### Beispiel 9

Hemmung der frühen Gravidität bei der Ratte:
Rattenweibchen werden im Prooestrus angepaart. Bei Nachweis von Spermien im Vaginalabstrich am nächsten Tag wird dieser Tag als Tag 1 (=d1) der Gravidität gerechnet. Die Behandlung mit Testsubstanz oder Vehikel erfolgt von d5 - d7, die Autopsie erfolgt d9. Die Substanzen werden in 0,2 ml Vehikel (Benzylbenzoat/Rizinusöl 1 + 4) subcutan injiziert. Die Rate vollständig gehemmter Graviditäten in den verschiedenen Gruppen ergibt sich aus Tabelle 1. Es wurde für J 867 eine dem RU 486 um den Faktor 3 überlegene Nidationshemmung festgestellt.

### Beispiel 10

Antigestagen-Behandlung weiblicher Meerschweinchen:

Behandlung von adulten weiblichen Meerschweinchen von Tag 10 bis Tag 18 des Zyklus (Autopsie). Applikation der angegebenen Dosen mittels subcutaner osmotischer Pumpen (ALZET Typ 2 ML1) Vehikel: 2,0 ml Propylenglykol/24 Std.

### Beispiel 11

### Antiglucocorticoide Wirkung von J867 in der humanen Brustkrebszellinie ZR75/AGP-763

Die Zellkulturenexperimente wurden in RPMI 1640, versetzt mit 10% fötalem Kälberserum (FKS) im Brutschrank mit 95% Luft und 5% CO₂ bei 37°C durchgeführt. Für die Experimente wurden die Zellen in 60 mm Petrischalen ausgesät. Das Medium der konfluenten Zellen wurde ersetzt durch ein Medium mit 5% FKS (behandelt mit dextran coated charcoal, DCC). Zur CAT-Induktion wurde Dexamethason in einer Konzentration von 10⁻⁷ M in Ethanol (0,2%; v/v) zugegeben. Die Ernte der stimulierten Zellen erfolgte 16 Stunden später durch Herstellung eines Zellextrakts mit Zellysispuffer. Die CAT-Bestimmung wurde mit einem ELISA der Firma Boehringer entsprechend der Beschreibung für die quantitative CAT-Bestimmung von transfizierten Zellen durchgeführt.

### Beispiel 12

Antiglucocorticoide Wirkung von J867 im TAT-Modell in Rattenhepatomzellen:
Die Zellkulturen wurden in DMEM unter ansonsten gleichen Bedingungen wie nach Beispiel 11 durchgeführt, Die Einsaat der Zellen für die Experimente erfolgte in 24 well Platten. Die Substanzzugabe zu den konfluenten Hepatomzellen erfolgte in 0,2% (v/v) Ethanol für 16 Stunden. Nach schonender Zellernte mit einem Zellschaber und Zellextraktgewinnung mittels Ultraschall wurde die TAT-Enzymbestimmung nach Diamondstone durchgeführt.

## Patentansprüche

1. 11β-Benzaldoxim-estra-4,9-dien-Derivate der allgemeinen Formel I, 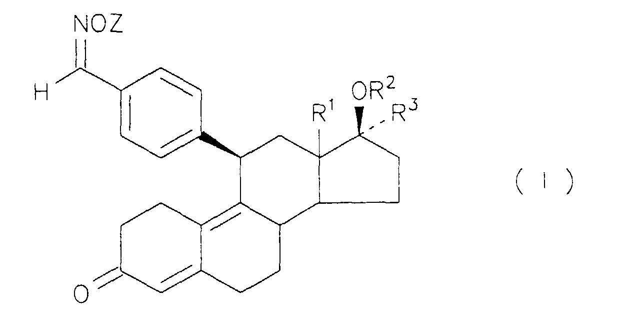 worin
R¹ ein Wasserstoffatom oder ein Alkylrest mit 1-6 Kohlenstoffatomen ist,
R² für ein Wasserstoffatom, eine Alkyl-, Aryl-, Ar-alkyl- oder Alkylarylgruppe mit 1-10 Kohlenstoffatomen, einen Acylrest mit 1-10 Kohlenstoffatomen oder einen Rest -CONHR⁴ oder -COOR⁴ steht,
wobei R⁴ ein Wasserstoffatom, einen Alkyl-, Aryl-, Aralkyl- oder Alkylarylrest mit 1-10 Kohlenstoffatomen bedeutet,
R³ ein Wasserstoffatom, eine Alkyl-, Aryl-,Aralkyl-oder Alkylarylgruppe mit 1 - 10 Kohlenstoffatomen,
einen Rest -(CH₂)ₙ-CH₂X,
wobei n = 0,1 oder 2 ist, X für ein Fluor-, Chlor-, Brom- oder Iodatom, für eine Cyano-, Azido- oder Rhodanogruppe, für einen Rest OR⁵ oder SR⁵ steht,
wobei R⁵ ein Wasserstoffatom, ein Alkyl-, Aryl-Aralkyl- oder Alkylarylrest mit 1-10 Kohlenstoffatomen oder ein Acylrest mit 1-10 Kohlenstoffatomen ist,
einen Rest OR⁵,
wobei R⁵ die oben angegebene Bedeutung hat,
einen Rest -(CH₂)ₒ-CH=CH(CH₂)ₚ-R⁶,
wobei o = 0, 1, 2 oder 3 und p = 0, 1 oder 2 ist und R⁶ für ein Wasserstoffatom, eine Alkyl-, Aryl-, Aralkyl- oder Alkylarylgruppe mit 1-10 Kohlenstoffatomen, eine Hydroxylgruppe, eine Alkoxygruppe oder Acyloxygruppe mit 1 - 10 Kohlenstoffatomen steht,
einen Rest -(CH₂)_{q}C≡CR⁷,
wobei q = 0, 1 oder 2 und R⁷ ein Wasserstoffatom, ein Fluor-, Chlor-, Brom- oder Iodatom,
ein Alkyl-, Aryl-, Aralkyl- oder Alkylarylrest mit 1-10 Kohlenstoffatomen oder ein Acylrest mit 1-10 Kohlenstoffatomen ist,
oder für den Fall, daß Z -H, R¹-CH₃, und R²-H oder -CH₃ ist, einen Rest -C≡C-CH₂OH bedeutet,
Z für ein Wasserstoffatom, einen Alkyl-, Aryl-, Aralkyl- oder Alkylarylrest mit 1-10 Kohlenstoffatomen, einen Acylrest mit 1-10 Kohlenstoffatomen, einen Rest -CONHR⁴ oder -COOR⁴,
wobei R⁴ die obenangegebene Bedeutung hat, oder für ein Alkali- oder Erdalkalimetallatom steht,
sowie deren pharmazeutisch annehmbaren Salze,
mit Ausnahme der Verbindungen, bei denen, R¹-CH₃, R²-CH₃ und R³-CH₂-O-CH₃ ist und Z für -CO-CH₃, -CO-O-C₂H₅, -CO-NH-Phenyl, -CO-NH-C₂H₅, -CO-C₂H₅ oder -CO-Phenyl steht.

2. Verbindungen nach Anspruch 1, dadurch gekennzeichnet, daß R¹ eine Methyl- oder Ethylgruppe ist.

3. Verbindungen nach mindestens einem der Ansprüche 1 und 2, dadurch gekennzeichnet, daß
R² für ein Wasserstoffatom, eine Alkylgruppe mit 1-10 Kohlenstoffatomen, einen Acylrest mit 1-10 Kohlenstoffatomen oder
einen Rest -CONHR⁴ oder -COOR⁴ steht,
wobei R⁴ ein Wasserstoffatom, einen Alkyl- oder Arylrest mit 1-10 Kohlenstoffatomen bedeutet.

4. Verbindungen nach Anspruch 3, dadurch gekennzeichnet, daß
R² für eine Alkylgruppe mit 1-6 Kohlenstoffatomen, einen Acylrest mit 1-6 Kohlenstoffatomen oder einen Rest -CONHR⁴ oder -COOR⁴ steht,
wobei R⁴ ein Wasserstoffatom, einen Alkyl- oder Arylrest mit 1-6 Kohlenstoffatomen bedeutet.

5. Verbindungen nach mindestens einem der Ansprüche 1 bis 4, dadurch gekennzeichnet, daß R³ ein Wasserstoffatom ist.

6. Verbindungen nach mindestens einem der Ansprüche 1 bis 4, dadurch gekennzeichnet, daß
R³ eine Alkyl-, Aryl-, Aralkyl- oder Alkylarylgruppe mit 1-10 Kohlenstoffatomen darstellt.

7. Verbindungen nach Anspruch 6, dadurch gekennzeichnet, daß
R³ für eine Alkylgruppe mit 1-10 Kohlenstoffatomen steht.

8. Verbindungen nach Anspruch 6, dadurch gekennzeichnet, daß
R³ für eine Alkylgruppe mit 1-6 Kohlenstoffatomen steht.

9. Verbindungen nach mindestens einem der Ansprüche 1 bis 4, dadurch gekennzeichnet, daß
R³ einen Rest -(CH₂)ₙ-CH₂X darstellt,
wobei n = 0,1 oder 2 ist, X für ein Fluor-, Chlor-, Brom- oder Iodatom, für eine Cyano-, Azido- oder Rhodanogruppe, einen Rest OR⁵ oder SR⁵ steht,
wobei R⁵ ein Wasserstoffatom, ein Alkylrest mit 1-10 Kohlenstoffatomen oder ein Acylrest mit 1-10 Kohlenstoffatomen ist.

10. Verbindungen nach Anspruch 9, dadurch gekennzeichnet, daß
R³ einen Rest -(CH₂)ₙ-CH₂X darstellt,
wobei n = 0,1 oder 2 ist, X für ein Fluor-, Chlor-, Brom- oder Iodatom, für eine Cyano-, Azido oder Rhodanogruppe, für einen Rest OR⁵ oder SR⁵ steht,
wobei R⁵ ein Alkylrest mit 1-6 Kohlenstoffatomen oder ein Acylrest mit 1-6 Kohlenstoffatomen ist.

11. Verbindungen nach mindestens einem der Ansprüche 1 bis 4, dadurch gekennzeichnet, daß
R³ einen Rest OR⁵ darstellt,
wobei R⁵ ein Wasserstoffatom, ein Alkylrest mit 1-10 Kohlenstoffatomen oder ein Acylrest mit 1-10 Kohlenstoffatomen ist.

12. Verbindungen nach Anspruch 11, dadurch gekennzeichnet, daß
R³ einen Rest OR⁵ darstellt,
wobei R⁵ ein Alkylrest mit 1-6 Kohlenstoffatomen oder ein Acylrest mit 1-6 Kohlenstoffatomen ist.

13. Verbindungen nach mindestens einem der Ansprüche 1 bis 4, dadurch gekennzeichnet, daß
R³ einen Rest -(CH₂)ₒ-CH=CH(CH₂)ₚ-R⁶ darstellt,
wobei o = 0, 1, 2 oder 3 und p = 0,1 oder 2 ist und R⁶ für ein Wasserstoffatom, eine Alkylgruppe mit 1-10 Kohlenstoffatomen, eine Hydroxylgruppe, eine Alkoxygruppe oder Acyloxygruppe mit 1 - 10 Kohlenstoffatomen steht.

14. Verbindungen nach Anspruch 13, dadurch gekennzeichnet, daß
R³ einen Rest -(CH₂)ₒ-CH=CH(CH₂)ₚ-R⁶ darstellt,
wobei o = 0, 1, 2 oder 3 und p = 0,1 oder 2 ist und R⁶ für eine Alkylgruppe mit 1-6 Kohlenstoffatomen, eine Alkoxygruppe oder Acyloxygruppe mit 1-6 Kohlenstoffatomen steht.

15. Verbindungen nach mindestens einem der Ansprüche 1 bis 4, dadurch gekennzeichnet, daß
R³ einen Rest-(CH₂)_{q}C≡CR⁷ darstellt,
wobei q = 0, 1 oder 2 und R⁷ ein Wasserstoffatom, ein Fluor-, Chlor-, Brom- oder Iodatom, ein Alkylrest mit 1-10 Kohlenstoffatomen oder ein Acylrest mit 1-10 Kohlenstoffatomen ist.

16. Verbindungen nach Anspruch 15, dadurch gekennzeichnet, daß
R³ einen Rest-(CH₂)_{q}C≡CR⁷ darstellt,
wobei q = 0, 1 oder 2 und R⁷ ein Alkylrest mit 1-6 Kohlenstoffatomen oder ein Acylrest mit 1-6 Kohlenstoffatomen ist.

17. Verbindungen nach mindestens einem der Ansprüche 1 bis 16, dadurch gekennzeichnet, daß
Z für ein Wasserstoffatom, einen Alkylrest mit 1-10 Kohlenstoffatomen, einen Acylrest mit 1-10 Kohlenstoffatomen, einen
Rest -CONHR⁴ oder -COOR⁴ steht,
wobei R⁴ ein Wasserstoffatom oder einen Alkylrest mit 1-10 Kohlenstoffatomen bedeutet.

18. Verbindungen nach Anspruch 17, dadurch gekennzeichnet, daß
Z für einen Alkylrest mit 1-6 Kohlenstoffatomen, einen Acylrest mit 1-6 Kohlenstoffatomen, einen
Rest -CONHR⁴ oder -COOR⁴ steht,
wobei R⁴ ein Wasserstoffatom, einen Alkyl- oder Arylrest mit 1-6 Kohlenstoffatomen bedeutet.

19. Verbindungen nach mindestens einem der Ansprüche 1 bis 16, dadurch gekennzeichnet, daß
Z für ein Alkali- oder Erdalkalimetallatom steht.

20. Verbindungen nach Anspruch 1, nämlich
11β-[4-(Hydroximinomethyl)phenyl]-17β-hydroxy-17α-methoxymethyl-estra-4,9-dien-3-on,
11β-[4-(Hydroximinomethyl)phenyl]-17β-hydroxy-17α-ethoxymethyl-estra-4,9-dien-3-on,
11β-[4-(Hydroximinomethyl)phenyl]-17β-hydroxy-17α-n-propoxymethyl-estra-4,9-dien-3-on,
11β-[4-(Hydroximinomethyl)phenyl]-17β-hydroxy-17α-1-propoxymethyl-estra-4,9-dien-3-on,
11β-[4-(Hydroximinomethyl)phenyl]-17β-methoxy-17α-methoxymethyl-estra-4,9-dien-3-on,
11β-[4-(Hydroximinomethyl)phenyl]-17β-methoxy-17α-ethoxymethyl-estra-4,9-dien-3-on,
11β-[4-(Hydroximinomethyl)phenyl]-17β-hydroxy-17α-(3-hydroxyprop-1-in-yl)-estra-4,9-dien-3-on,
11β-[4-(Hydroximinomethyl)phenyl]-17β-methoxy-17α-(3-hydroxyprop-1-in-yl)-estra-4,9-dien-3-on,
11β-[4-(Hydroximinomethyl)phenyl]-17β-hydroxy-17α-Z-(3-hydroxypropenyl)-estra-4,9-dien-3-on,
11β-[4-(Hydroximinomethyl)phenyl]-17β-methoxy-17α-Z-(3-hydroxypropenyl)-estra-4,9-dien-3-on,
17α-Chlormethyl-11β-[4-(hydroximinomethyl)phenyl]-17β-hydroxy-estra-4,9-dien-3-on,
17α-Chlormethyl-11β-[4-(hydroximinomethyl)phenyl]-17β-methoxy-estra-4,9-dien-3-on,
17α-Cyanomethyl-11β-[4-(hydroximinomethyl)phenyl]-17β-hydroxy-estra-4,9-dien-3-on,
17α-Cyanomethyl-11β-[4-(hydroximinomethyl)phenyl]-17β-methoxy-estra-4,9-dien-3-on,
17α-Azidomethyl-11β-[4-(hydroximinomethyl)phenyl]-17β-methoxy-estra-4,9-dien-3-on,
11β-[4-(Hydroximinomethyl)phenyl]-17β-methoxy-17α-methylthiomethyl-estra-4,9-dien-3-on,
11β-[4-(Methyloximinomethyl)phenyl]-17β-methoxy-17α-methoxymethyl-estra-4,9-dien-3-on,
11β-[4-(Methyloximinomethyl)phenyl]-17β-hydroxy-17α-methoxymethyl-estra-4,9-dien-3-on,
11β-[4-(Hydroximinomethyl)phenyl]-17β-ethoxy-17α-ethoxymethyl-estra-4,9-dien-3-on.

21. Verfahren zur Herstellung der Verbindungen nach Anspruch 1 und deren pharmazeutisch annehmbaren Salzen, dadurch gekennzeichnet, daß man eine Verbindung der allgemeinen Formel II, 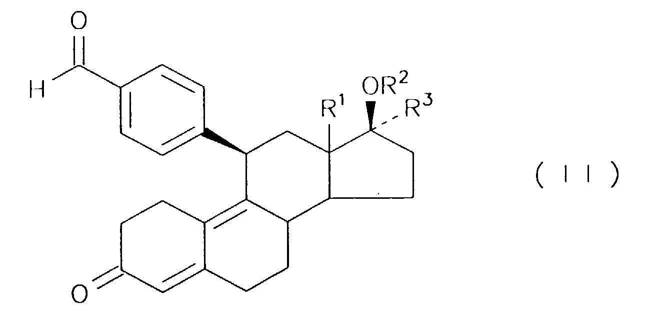 worin R¹, R² und R³ die in Anspruch 1 angegebene Bedeutung haben mit einer Verbindung der allgemeinen Formel IIa
NH₂-O-Y (IIa)
worin Y ein Wasserstoffatom, ein Alkylrest mit 1-10 Kohlenstoffatomen, ein Acylrest mit 1-10 Kohlenstoffatomen oder ein
Rest -CONHR⁴ oder -COOR⁴ ist,
wobei R⁴ die in Anspruch 1 angegebene Bedeutung hat,
und wobei die Verbindung der allgemeinen Formel IIa gegebenfalls in Form einer solchen Verbindung vorliegt, oder aus der die Verbindung der allgemeinen Formel IIa unter den gewählten Reaktionsbedingungen freigesetzt wird,
umsetzt, die gegebenenfalls vorhandene Hydroximinogruppe zu Verbindungen der Formel I, in denen Z Kein H ist, verestert oder verethert und gegebenenfalls die erhaltene Verbindung in ein pharmazeutisch annehmbares Salz überführt.

22. Verfahren nach Anspruch 21, dadurch gekennzeichnet, daß man die Verbindungen der allgemeinen Formel II und der allgemeinen Formel IIa in äquimolaren Mengen umsetzt.

23. Pharmazeutische Zusammensetzungen, gekennzeichnet durch den Gehalt an mindestens einer Verbindung nach einem der Ansprüche 1 bis 20.

## Claims

1. 11β-benzaldoxime-estra-4,9-derivatives of the general formula I 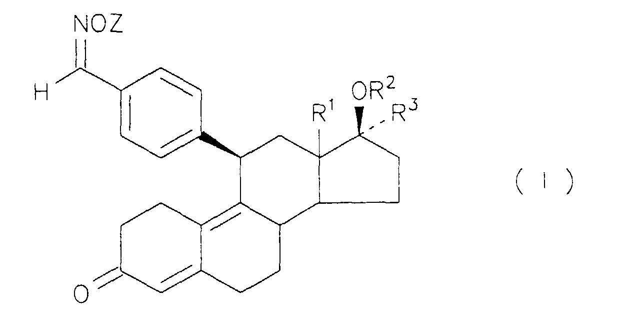 where
R¹ is a hydrogen atom or alkyl residue containing 1-6 carbon atoms,
R² is a hydrogen atom, an alkyl, aryl, aralkyl, or alkylaryl group containing 1-10 carbon atoms, an acyl residue containing 1-10 carbon atoms, or a residue -CONHR⁴ or -COO^{R4},
where R⁴ is a hydrogen atom, an alkyl, aryl, aralkyl, or alkylaryl residue containing 1-10 carbon atoms,
R³ is a hydrogen atom, an alkyl, aryl, aralkyl, or alkylaryl group containing 1-10 carbon atoms,
a residue -(CH₂)ₙ-CH₂X,
where n = 0, 1, or 2, X represents a fluorine, chlorine, bromine, or iodine atom, a cyano, acido, or rhodano group, a residue OR⁵ or SR⁵,
R⁵ being a hydrogen atom, an alkyl, aryl, aralkyl, or alkylaryl residue containing 1-10 carbon atoms, or an acyl residue containing 1-10 carbon atoms,
a residue OR⁵,
in which R5 has the meaning specified above,
a residue -(CH₂)ₒ-CH=CH(CH₂)ₚ-R⁶,
where o = 0, 1, 2, or 3 and p = 0, 1, or 2, and R⁶ represents a hydrogen atom, an alkyl, aryl, aralkyl, or alkylaryl group containing 1-10 carbon atoms, a hydroxyl group, an alkoxy or acyloxy group containing 1-10 carbon atoms,
a residue -(CH₂)_{q}C≡CR⁷,
where q = 0, 1, or 2, and R⁷ represents a hydrogen atom, a fluorine, chlorine, bromine, or iodine atom, an alkyl, aryl, aralkyl, or alkylaryl residue containing 1-10 carbon atoms, or an acyl residue containing 1-10 carbon atoms,
or, in case that Z is -H, R¹ is -CH₃ and R² is -H or - CH₃, represents a residue -C≡C-CH₂OH,
Z represents a hydrogen atom, an alkyl, aryl, aralkyl, or alkylaryl residue containing 1-10 carbon atoms, or an acyl residue containing 1-10 carbon atoms, a residue
-CONHR⁴ or -COOR⁴,
where R⁴ has the meaning specified above, or an alkaline or alkaline-earth metal atom,
and their pharmaceutically acceptable salts,
with the exception of compounds in which R¹ is -CH₃, R² is -CH₃, R³ is -CH₂-O-CH₃, and Z represents -CO-CH₃, -CO-O-C₂H₅, -CO-NH-phenyl, -CO-NH-C₂H₅, -CO-C₂H₅ or -CO-phenyl.

2. Compounds according to Claim 1, characterized in that
R¹ is a methyl or ethyl group.

3. Compounds according to at least one of Claims 1 and 2, characterized in that
R² represents a hydrogen atom, an alkyl group containing 1-10 carbon atoms, an acyl residue containing 1-10 carbon atoms, or a residue -CONHR⁴ or -COOR⁴,
where R⁴ is a hydrogen atom, an alkyl or aryl residue containing 1-10 carbon atoms.

4. Compounds according to Claim 3, characterized in that
R² represents an alkyl group containing 1-6 carbon atoms, an acyl residue containing 1-6 carbon atoms, or
a residue -CONHR⁴ or -COOR⁴,
where R⁴ is a hydrogen atom or an alkyl or aryl residue containing 1-6 carbon atoms.

5. Compounds according to at least one of Claims 1 to 4, characterized in that R³ is a hydrogen atom.

6. Compounds according to at least one of Claims 1 to 4, characterized in that R³ is an alkyl, aryl, aralkyl, or alkylaryl group containing 1-10 carbon atoms.

7. Compounds according to Claim 6, characterized in that
R³ represents an alkyl group containing 1-10 carbon atoms.

8. Compounds according to Claim 6, characterized in that
R³ represents an alkyl group containing 1-6 carbon atoms.

9. Compounds according to at least one of Claims 1 to 4, characterized in that
R³ is a a residue -(CH₂)ₙ-CH₂X,
where n = 0, 1, or 2, X represents a fluorine, chlorine, bromine, or iodine atom, a cyano, acido, or rhodano group, a residue OR⁵ or SR⁵,
R⁵ being a hydrogen atom, an alkyl residue containing 1-10 carbon atoms, or an acyl residue containing 1-10 carbon atoms.

10. Compounds according to Claim 9, characterized in that
R³ is a a residue -(CH₂)ₙ-CH₂X,
where n = 0, 1, or 2, X represents a fluorine, chlorine, bromine, or iodine atom, a cyano, acido, or rhodano group, a residue OR⁵ or SR⁵,
R⁵ being an alkyl residue containing 1-6 carbon atoms, or an acyl residue containing 1-6 carbon atoms.

11. Compounds according to at least one of Claims 1 to 4, characterized in that
R³ represents a residue OR⁵,
R⁵ being a hydrogen atom, an alkyl residue containing 1-10 carbon atoms, or an acyl residue containing 1-10 carbon atoms.

12. Compounds according to Claim 11, characterized in that
R³ represents a residue OR⁵,
R⁵ being an alkyl residue containing 1-6 carbon atoms, or an acyl residue containing 1-6 carbon atoms.

13. Compounds according to at least one of Claims 1 to 4, characterized in that
R³ represents a residue -(CH₂)ₒ-CH=CH(CH₂)ₚ-R⁶,
where o = 0, 1, 2, or 3 and p = 0, 1, or 2, and R⁶ represents a hydrogen atom, an alkyl group containing 1-10 carbon atoms, a hydroxyl group, an alkoxy or acyloxy group containing 1-10 carbon atoms.

14. Compounds according to Claim 13, characterized in that
R³ represents a residue -(CH₂)ₒ-CH=CH(CH₂)ₚ-R⁶,
where o = 0, 1, 2, or 3 and p = 0, 1, or 2, and R⁶ represents an alkyl group containing 1-6 carbon atoms, an alkoxy or acyloxy group containing 1-6 carbon atoms.

15. Compounds according to at least one of Claims 1 to 4, characterized in that
R³ represents a a residue -(CH₂)_{q}C≡CR⁷,
where q = 0, 1, or 2, and R⁷ represents a hydrogen atom, a fluorine, chlorine, bromine, or iodine atom, an alkyl residue containing 1-10 carbon atoms, or an acyl residue containing 1-10 carbon atoms.

16. Compounds according to Claim 15, characterized in that
R³ represents a a residue -(CH₂)_{q}C≡CR⁷,
where q = 0, 1, or 2, and R⁷ represents an alkyl residue containing 1-6 carbon atoms, or an acyl residue containing 1-6 carbon atoms.

17. Compounds according to at least one of Claims 1 to 16, characterized in that
Z represents a hydrogen atom, an alkyl residue containing
1-10 carbon atoms, an acyl residue containing 1-10 carbon atoms, a residue -CONHR⁴ or -COOR⁴,
where R⁴ is a hydrogen atom or an alkyl residue containing 1-10 carbon atoms.

18. Compounds according to Claim 17, characterized in that
Z represents an alkyl residue containing 1-6 carbon atoms, an acyl residue containing 1-6 carbon atoms, a residue -CONHR⁴ or -COOR⁴,
where R⁴ is a hydrogen atom or an alkyl residue containing 1-6 carbon atoms.

19. Compounds according to at least one of Claims 1 to 16, characterized in that
Z represents an alkaline or alkaline-earth metal atom.

20. Compounds according to Claim 1, namely
11β-[4-(hydroximinomethyl)phenyl]-17β-hydroxy-17α-methoxymethyl-estra-4,9-diene-3-on,
11β-[4-(hydroximinomethyl)phenyl]-17β-hydroxy-17α-ethoxymethyl-estra-4,9-diene-3-on,
11β-[4-(hydroximinomethyl)phenyl]-17β-hydroxy-17α-n-propoxymethyl-estra-4,9-diene-3-on,
11β-[4-(hydroximinomethyl)phenyl]-17β-hydroxy-17α-i-propoxymethyl-estra-4,9-diene-3-on,
11β-[4-(hydroximinomethyl)phenyl]-17β-methoxy-17α-methoxymethyl-estra-4,9-diene-3-on,
11β-[4-(hydroximinomethyl)phenyl]-17β-methoxy-17α-ethoxymethyl-estra-4,9-diene-3-on,
11β-[4-(hydroximinomethyl)phenyl]-17β-hydroxy-17α-(3-hydroxyprop-1-in-yl)-estra-4,9-diene-3-on,
11β-[4-(hydroximinomethyl)phenyl]-17β-methoxy-17α-(3-hydroxyprop-1-in-yl)-estra-4,9-diene-3-on,
11β-[4-(hydroximinomethyl)phenyl]-17β-hydroxy-17α-Z-(3-hydroxypropenyl)-estra-4,9-diene-3-on,
11β-[4-(hydroximinomethyl)phenyl]-17β-methoxy-17α-Z-(3-hydroxypropenyl)-estra-4,9-diene-3-on,
17α-chloromethane-11β-[4-(hydroximinomethyl)phenyl]-17β-hydroxy-estra-4,9-diene-3-on,
17α-chloromethane-11β-[4-(hydroximinomethyl)phenyl]-17β-methoxy-estra-4,9-diene-3-on,
17α-cyanomethyl-11β-[4-(hydroximinomethyl)phenyl]-17β-hydroxy-estra-4,9-diene-3-on,
17α-cyanomethyl-11β-[4-(hydroximinomethyl)phenyl]-17β-methoxy-estra-4,9-diene-3-on,
17α-acidomethyl-11β-[4-(hydroximinomethyl)phenyl]-17β-methoxy-estra-4,9-diene-3-on,
11β-[4-(hydroximinomethyl)phenyl]-17β-methoxy-17α-methylthiomethyl-estra-4,9-diene-3-on,
11β-[4-(methyloximinomethyl)phenyl]-17β-methoxy-17α-methoxymethyl-estra-4,9-diene-3-on,
11β-[4-(methyloximinomethyl)phenyl]-17β-hydroxy-17α-methoxymethyl-estra-4,9-diene-3-on, and
11β-[4-(hydroximinomethyl)phenyl]-17β-ethoxy-17α-ethoxymethyl-estra-4,9-diene-3-on.

21. Method for producing the compounds according to Claim 1 and their pharmaceutically acceptable salts,
characterized in that a compound of general formula II 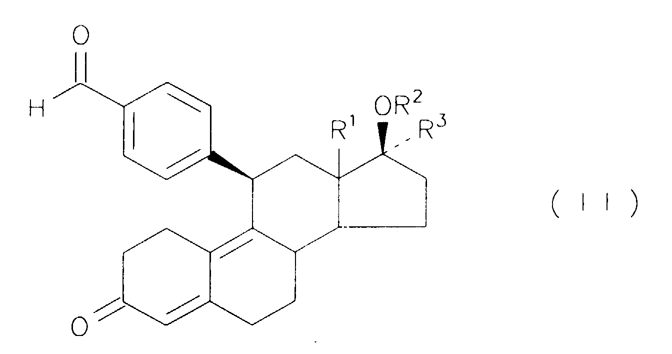 wherein
R¹, R² and R³ have the meaning as specified in claim 1, is reacted with a compound of the general formula IIa
NH2-O-Y (IIa)
where Y is a hydrogen atom, an alkyl residue containing 1-10 carbon atoms, an acyl residue containing 1-10 carbon atoms, or a residue -CONHR⁴ or -COOR⁴,
where R⁴ has the meaning as specified in claim 1,
and where the compound of the general formula IIa is present, if required, in the form of such compound, or from which the compound of the general formula IIa is released under the selected conditions of the reaction,
the hydroximino group, if present, is esterified or etherified to produce compounds of the formula I, in which Z is not -H, and the resulting compound is optionally salified into its pharmaceutically acceptable salt.

22. Method according to Claim 21, characterized in that the compounds of general formula II are reacted with the compounds of general formula IIa in equimolar quantities.

23. Pharmaceutical compositions, characterized in that they contain at least one compound according to one of Claims 1 to 20.

## Revendications

1. Dérivés du 11β-benzaldoxime-estra-4,9-diène de formule générale I 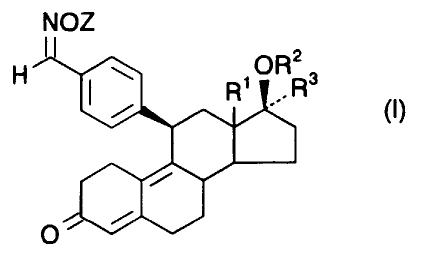 dans laquelle
R¹ est un atome d'hydrogène ou un reste alkyle à 1 à 6 atomes de carbone,
R² représente un atome d'hydrogène, un groupe alkyle, aryle, aralkyle ou alkylaryle à 1 à 10 atomes de carbone, un reste acyle à 1 à 10 atomes de carbone ou un reste -CONHR⁴ ou -COOR⁴,
dans lequel R⁴ signifie un atome d'hydrogène, un reste alkyle, aryle, aralkyle ou alkylaryle à 1 à 10 atomes de carbone,
R³ signifie un atome d'hydrogène, un groupe alkyle, aryle, aralkyle ou alkylaryle à 1 à 10 atomes de carbone,
un reste -(CH₂)ₙ-CH₂X,
dans lequel n = 0, 1 ou 2, X représente un atome de fluor, de chlore, de brome ou d'iode, un groupe cyano, azido ou rhodano, un reste OR⁵ ou SR⁵,
dans lequel R⁵ est un atome d'hydrogène, un reste alkyle, aryle, aralkyle ou alkylaryle à 1 à 10 atomes de carbone ou un reste acyle à 1 à 10 atomes de carbone,
un reste OR⁵,
dans lequel R⁵ a les significations données ci-dessus,
un reste -(CH₂)ₒ-CH=CH(CH₂)ₚ-R⁶,
dans lequel o = 0, 1, 2 ou 3 et p = 0, 1 ou 2 et R⁶ représente un atome d'hydrogène, un groupe alkyle, aryle, aralkyle ou alkylaryle à 1 à 10 atomes de carbone, un groupe hydroxyle, un groupe alcoxy ou un groupe acyloxy à 1 à 10 atomes de carbone,
un reste -(CH₂)_{q}C≡CR⁷,
dans lequel q = 0, 1 ou 2 et R⁷ représente un atome d'hydrogène, un atome de fluor, de chlore, de brome ou d'iode, un reste alkyle,
aryle, aralkyle ou alkylaryle à 1 à 10 atomes de carbone ou un reste acyle à 1 à 10 atomes de carbone,
ou un reste -C≡C-CH₂OH, dans le cas où Z représente -H, R¹ représente -CH₃ et R² représente -H ou -CH₃,
Z représente un atome d'hydrogène, un reste alkyle, aryle, aralkyle ou alkylaryle à 1 à 10 atomes de carbone, un reste acyle à 1 à 10 atomes de carbone, un reste -CONHR⁴ ou -COOR⁴,
dans lequel R⁴ a la signification donnée ci-dessus,
ou un atome de métal alcalin ou alcalino-terreux,
ainsi que leurs sels pharmaceutiquement acceptables,
à l'exception des composés dans lesquels R¹ est un groupe -CH₃, R² est un groupe -CH₃, et R³ est un groupe -CH₂-O-CH₃ et Z représente -CO-CH₃, -CO-O-C₂H₅, -CO-NH-phényl, -CO-NH-C₂H₅, -CO-C₂H₅ ou -CO-phényl.

2. Composés selon la revendication 1, caractérisés en ce que R¹ est un groupe méthyle ou éthyle.

3. Composés selon au moins une des revendications 1 ou 2, caractérisé en ce que
R² représente un atome d'hydrogène, un groupe alkyle à 1 à 10 atomes de carbone, un reste acyle à 1 à 10 atomes de carbone ou un reste -CONHR⁴ ou -COOR⁴,
dans lequel R⁴ signifie un atome d'hydrogène, un reste alkyle ou aryle à 1 à 10 atomes de carbone.

4. Composés selon la revendication 3, caractérisés en ce que
R² représente un groupe alkyle à 1 à 6 atomes de carbone, un groupe acyle à 1 à 6 atomes de carbone ou un reste -CONHR⁴ ou -COOR⁴,
dans lequel R⁴ signifie un atome d'hydrogène, un reste alkyle ou aryle à 1 à 6 atomes de carbone.

5. Composés selon au moins une des revendications 1 à 4, caractérisés en ce que R³ est un atome d'hydrogène.

6. Composés selon au moins une des revendications 1 à 4, caractérisés en ce que
R³ représente un groupe alkyle, aryle, aralkyle ou alkylaryle à 1 à 10 atomes de carbone.

7. Composés selon la revendication 6, caractérisés en ce que
R³ représente un groupe alkyle à 1 à 10 atomes de carbone.

8. Composés selon la revendication 6, caractérisés en ce que
R³ représente un groupe alkyle à 1 à 6 atomes de carbone.

9. Composés selon au moins une des revendications 1 à 4, caractérisés en ce que
R³ représente un reste -(CH₂)ₙ-CH₂X,
dans lequel n = 0, 1 ou 2, X représente un atome de fluor, de chlore, de brome ou d'iode, un groupe cyano, azido ou rhodano, un reste OR⁵ ou SR⁵,
dans lequel R⁵ est un atome d'hydrogène, un reste alkyle à 1 à 10 atomes de carbone ou un reste acyle à 1 à 10 atomes de carbone.

10. Composés selon la revendication 9, caractérisés en ce que
R³ représente un reste -(CH₂)ₙ-CH₂X,
dans lequel n = 0, 1 ou 2, X représente un atome de fluor, de chlore, de brome ou d'iode, un groupe cyano, azido ou rhodano, un reste OR⁵ ou SR⁵,
dans lequel R⁵ est un reste alkyle à 1 à 6 atomes de carbone ou un reste acyle à 1 à 6 atomes de carbone.

11. Composés selon au moins une des revendications 1 à 4, caractérisés en ce que
R³ représente un reste OR⁵,
dans lequel R⁵ est un atome d'hydrogène, un reste alkyle à 1 à 10 atomes de carbone ou un reste acyle à 1 à 10 atomes de carbone.

12. Composés selon la revendication 11, caractérisés en ce que R³ représente un reste OR⁵,
dans lequel R⁵ est un reste alkyle à 1 à 6 atomes de carbone ou un reste acyle à 1 à 6 atomes de carbone.

13. Composés selon au moins une des revendications 1 à 4, caractérisés en ce que
R³ représente un reste -(CH₂)ₒ-CH=CH(CH₂)ₚ-R⁶,
dans lequel o = 0, 1, 2 ou 3 et p = 0, 1 ou 2 et R⁶ représente un atome d'hydrogène, un groupe alkyle à 1 à 10 atomes de carbone, un groupe hydroxyle, un groupe alcoxy ou un groupe acyloxy à 1 à 10 atomes de carbone.

14. Composés selon la revendication 13, caractérisés en ce que
R³ représente un reste -(CH₂)ₒ-CH=CH(CH₂)ₚ-R⁶,
dans lequel o = 0, 1, 2 ou 3 et p = 0, 1 ou 2 et R⁶ représente un groupe alkyle à 1 à 6 atomes de carbone, un groupe alcoxy ou un groupe acyloxy à 1 à 6 atomes de carbone.

15. Composés selon au moins une des revendications 1 à 4, caractérisés en ce que
R³ représente un reste -(CH₂)_{q}C≡CR⁷,
dans lequel q = 0, 1 ou 2 et R⁷ représente un atome d'hydrogène, un atome de fluor, de chlore, de brome ou d'iode, un reste alkyle à 1 à 10 atomes de carbone ou un reste acyle à 1 à 10 atomes de carbone.

16. Composés selon la revendication 15, caractérisés en ce que R³ représente un reste -(CH₂)_{q}C≡CR⁷,
dans lequel q = 0, 1 ou 2 et R⁷ représente un reste alkyle à 1 à 6 atomes de carbone ou un reste acyle à 1 à 6 atomes de carbone.

17. Composés selon une des revendications 1 à 16, caractérisés en ce que
Z représente un atome d'hydrogène, un reste alkyle à 1 à 10 atomes de carbone, un reste acyle à 1 à 10 atomes de carbone, un reste -CONHR⁴ ou -COOR⁴,
dans lequel R⁴ signifie un atome d'hydrogène ou un reste alkyle à 1 à 10 atomes de carbone.

18. Composés selon la revendication 17, caractérisés en ce que
Z représente un reste alkyle à 1 à 6 atomes de carbone, un reste acyle à 1 à 6 atomes de carbone, un reste -CONHR⁴ ou -COOR⁴,
dans lequel R⁴ signifie un atome d'hydrogène, un reste alkyle ou aryle à 1 à 6 atomes de carbone.

19. Composés selon une des revendications 1 à 16, caractérisés en ce que
Z représente un atome de métal alcalin ou alcalino-terreux.

20. Composés selon la revendication 1, à savoir les composés
11β-[4-(hydroximinométhyl)phényl]-17β-hydroxy-17α-méthoxyméthyl-estra-4,9-diène-3-one,
11β-[4-(hydroximinométhyl)phényl]-17β-hydroxy-17α-éthoxyméthyl-estra-4,9-diène-3-one,
11β-[4-(hydroximinométhyl)phényl]-17β-hydroxy-17α-*n*-propoxyméthyl-estra-4,9-diène-3-one,
11β-[4-hydroximinométhyl)phényl]-17β-hydroxy-17α-*i*-propoxyméthyl-estra-4,9-diène-3-one,
11β-[4-(hydroximinométhyl)phényl]-17β-méthoxy-17α-méthoxyméthyl-estra-4,9-diène-3-one,
11β-[4-(hydroximinométhyl)phényl]-17β-méthoxy-17α-éthoxyméthyl-estra-4,9-diène-3-one,
11β-[4-hydroximinométhyl)phényl]-17β-hydroxy-17α-(3-hydroxyprop-1-in-yl)estra-4,9-diéne-3-one,
11β-[4-hydroximinométhyl)phényl]-17β-méthoxy-17α-(3-hydroxyprop-1-in-yl)estra-4,9-diéne-3-one,
11β-[4-(hydroximinométhyl)phényl]-17β-hydroxy-17α-Z-(3-hydroxypropényl)estra-4,9-diéne-3-one,
11β-[4-hydroximinométhyl)phényl]-17β-méthoxy-17α-Z-(3-hydroxypropényl)estra-4,9-diéne-3-one,
17α-chlorométhyl-11β-[4-(hydroximinométhyl)phényl]-17β-hydroxy-estra-4,9-diéne-3-one,
17α-chlorométhyl-11β-[4-(hydroximinométhyl)phényl]-17β-méthoxy-estra-4,9-diène-3-one,
17α-cyanométhyl-11β-[4-(hydroximinométhyl)phényl)-17β-hydroxy-estra-4,9-diène-3-one,
17α-cyanométhyl-11β-[4-(hydroximinométhyl)phényl]-17β-méthoxy-estra-4,9-diène-3-one,
17α-azidométhyl-11β-[4-(hydroximinométhyl)phényl]-17β-méthoxy-estra-4,9-diéne-3-one,
11β-[4-(hydroximinométhyl)phényl]-17β-méthoxy-17α-méthylthiométhyl-estra-4,9-diène-3-one,
11β-[4-(méthyloximinométhyl)phényl]-17β-méthoxy-17α-méthoxyméthyl-estra-4,9-diène-3-one,
11β-[4-(méthyloximinométhyl)phényl]-17β-hydroxy-17α-méthoxyméthyl-estra-4,9-diéne-3-one,
11β-[4-(hydroximinométhyl)phényl]-17β-éthoxy-17α-éthoxyméthyl-estra-4,9-diène-3-one.

21. Procédé pour la préparation des composés selon la revendication 1 et de leurs sels pharmaceutiquement acceptables, caractérisé en ce que l'on fait réagir un composé de formule générale II 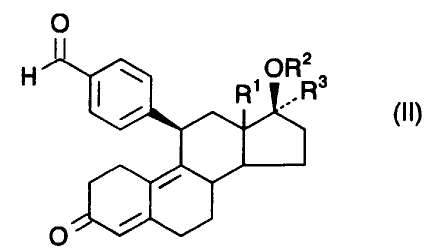 dans laquelle les groupes R¹, R² et R³ ont les significations données à la revendication 1,
avec un composé de formule générale IIa
NH₂-O-Y (IIa)
dans laquelle Y représente un atome d'hydrogène, un groupe alkyle à 1 à 10 atomes de carbone, un reste acyle à 1 à 10 atomes de carbone ou un reste -CONHR⁴ ou -COOR⁴,
dans lequel R⁴ a la signification donnée à la revendication 1,
et où le composé de formule générale IIa se présente éventuellement sous forme d'un tel composé ou bien d'un autre à partir duquel le composé de formule générale IIa est libéré dans les conditions de réaction choisies,
on estérifie ou éthérifie les groupes hydroximino éventuellement présents pour obtenir des composés de formule I dans laquelle Z n'est pas un atome d'hydrogène et on transforme éventuellement le composé obtenu en un sel pharmaceutiquement acceptable.

22. Procédé selon la revendication 21, caractérisé en ce que l'on fait réagir le composé de formule générale II et celui de formule générale IIa en quantités équimolaires.

23. Préparation pharmaceutique caractérisée en ce qu'elle contient au moins un composé selon une des revendications 1 à 20.
